(19) Europäisches Patentamt

European Patent Office

Office européen des brevets

(11) **EP 2 782 064 A1**

(12) **EUROPEAN PATENT APPLICATION**

(43) Date of publication:
24.09.2014 Bulletin 2014/39

(51) Int Cl.:
***G06Q 50/24*** *(2012.01)*

(21) Application number: 13159809.6

(22) Date of filing: 18.03.2013

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Optimal Medicine Ltd**
**London EC3V 3ND (GB)**

(72) Inventors:
• **Munro, Janet**
**34380 St Martin du Londres (FR)**

• **McBurney, robert**
**Chesnut Hill, MA 02467 (US)**
• **Turner, Rick**
**Manchester M1 6DE (GB)**
• **Tubman, Kenneth L**
**Millville, MA 01529 (US)**

(74) Representative: **Miller Sturt Kenyon**
**9 John Street**
**London WC1N 2ES (GB)**

(54) **Personalised medicine system**

(57)    A personalised medicine system comprising: a processor; a memory; an input module; and an output module, said personalised medicine system using a first database of side effects associated with at least one predetermined condition and of moderators influencing a likelihood of experiencing said side effects, and being adapted: to receive an input of patient data of a patient; and based on the patient data and the first database, to determine for each side effect a first risk indicator of a risk that the patient will experience the side effect.

A method and a computer program are also provided.

Fig. 22

EP 2 782 064 A1

## Description

## Field of the Invention

The present invention relates to a personalised medicine system, such as a system that provides clinical decision support and in particular relates to a system that provides an indication of the general risk of a patient experiencing a side effect and of the risk the patient will experience the side effect as a result of any particular medication.

## Background of the Invention

In general existing practice, it is not possible for clinicians to take account of the large numbers of different factors that affect the likelihood of a patient undergoing treatment experiencing side effects, particularly during a consultation, and clinicians rely mainly on their own experience in assessing the likelihood of side effects when prescribing medications. Moreover, the risk specific to a particular patient of experiencing side effects due to a particular drug is not considered during patient consultations, instead, the side effect risk profile of the particular drug is considered.

## Summary of the Invention

The present invention seeks to address these shortcomings by providing a personalised medicine system that displays an indication to a clinician and patient of the risks of a patient experiencing side effects, in general and as a result of taking particular medications.

According to a first aspect of the present invention, there is provided a personalised medicine system comprising: a processor; a memory; an input module; and an output module, said personalised medicine system using a first database of side effects associated with at least one predetermined condition and of moderators influencing a likelihood of experiencing said side effects, and being adapted: to receive an input of patient data of a patient; and based on the patient data and the first database, to determine for each side effect a first risk indicator of a risk that the patient will experience the side effect.

Preferably, the database includes, for each of said moderators, at least one category associated with the moderator, and each side effect associated with each category.

In this case, it is preferred that the database further stores a strength value for each moderator and a category value for each category.

It is further preferred that the determination of a said first risk indicator for a side effect comprises: establishing which categories influence a likelihood of experiencing the side effect; for each established category, determining the categories in which the patient falls based on the patient data; and combining the category value of each of the determined categories with the respective strength value of the corresponding moderator.

It is also further preferred that at least the category values for each moderator are normalised, whereby minimum and maximum allowed category values are the same for each moderator.

Preferably, the first risk indicator is an indication of a risk compared to a general population that the patient will experience the side effect.

Preferably, the system is further configured to display which moderators influence a likelihood of the patient experiencing a side effect.

Preferably, the system is further configured to display the side effects in order of the values of the respective first risk indicators.

Preferably, the system is further configured, on receipt of an input from a user, to store an indication that a patient wishes to avoid drugs increasing a likelihood of experiencing a displayed side effect.

Preferably, the system further uses a second database of side effects associated with each of a plurality of drugs and weightings influencing a likelihood of experiencing said effects for each drug, said system being adapted based on the first risk indicators and the weightings, to determine for each drug a second risk indicator of a risk that the patient will experience side effects from taking the drug.

According to a further aspect of the present invention, there is provided a personalised medicine system comprising: a processor; a memory; an input module; and an output module, said personalised medicine system using: for each of a plurality of side effects, a respective first indicator of a risk that a specific patient will experience the side effect; and a table of side effects associated with each of a plurality of drugs and weightings influencing a likelihood of experiencing said effects for each drug, and being adapted: based on the first indicators and the weightings, to determine for each drug a second indicator of a risk that the patient will experience side effects from taking the drug.

In either aspect, it is preferred that the second indicator for a drug is determined by combining the weighting for each respective side effect associated with the drug with first indicator for the corresponding side effect.

Preferably, the second indicator corresponds to the highest combination of a weighting with a respective first risk indicator.

It is further preferred that the second risk indicator corresponds to a combination of respective combinations weightings with first risk indicators.

Preferably, the system is further configured to: store an indication that the patient wishes to avoid a side effect; and determine the second indicator based on the indication.

Preferably, the system is further configured to display a list of drugs for use by the patient, together with a representation of the likelihood a patient will suffer a side effect associated with the drug.

Preferably, the system in this case is further configured to: store at least one of a diagnosis of an illness and symptoms experienced by the patient; and determine the list of drugs based on at least one of the diagnosis and the symptoms.

It is further preferred that the list comprises a predetermined number of drugs most commonly prescribed by a predetermined group.

Preferably, the representation is based on whether the second indicator for a drug is above a predetermined threshold.

**Brief Description of the Drawings**

Embodiments of the present invention will now be described by way of further example only and with reference to the accompanying drawings, in which:

Fig. 1 is a schematic diagram of the architecture of a device for use in the present invention;

Fig. 2 is a schematic diagram of a system according to the present invention;

Fig. 3 is a table representing a workflow for use in the present invention;

Fig. 4 is a partial representation of a screen for display in the present invention, including a header, another display page and a guidelines area;

Fig. 5 is a partial representation of an actions from last visit display page for use in the present invention;

Fig. 6 is a partial representation of an actions due display page for use in the present invention;

Fig. 7 is a partial representation of another display screen for use in the present invention, including two display pages and a guidelines area;

Fig. 8 is a partial representation of another display screen for use in the present invention, including a display page and a guidelines area;

Fig. 9 is a partial representation of another display screen for use in the present invention, including a header, a display page and a guidelines area;

Fig. 10 is a partial representation of another display screen for use in the present invention, including a header, a display page and a guidelines area;

Fig. 11 is a representation of a pop-up display for a symptom rating scale for use in the present invention;

Fig. 12 is a partial representation of a symptom severity sliders display page for use in the present invention;

Fig. 13 is a partial representation of side effect rating scale display page for use in the present invention;

Fig. 14 is a partial representation of another display screen for use in the present invention, including a header, a display page and a guidelines area;

Fig. 15 is a partial representation of a side effect severity sliders display page for use in the present invention;

Fig. 16 is a partial representation of another display screen for use in the present invention, including a header, a display page and a guidelines area;

Fig. 17 is a partial representation of another display screen for use in the present invention, including a header, a display page and a guidelines area;

Fig. 18 is a partial representation of a side effect relative risk display page for use in the present invention;

Fig. 19 is a partial representation of a drug information display page for use in the present invention;

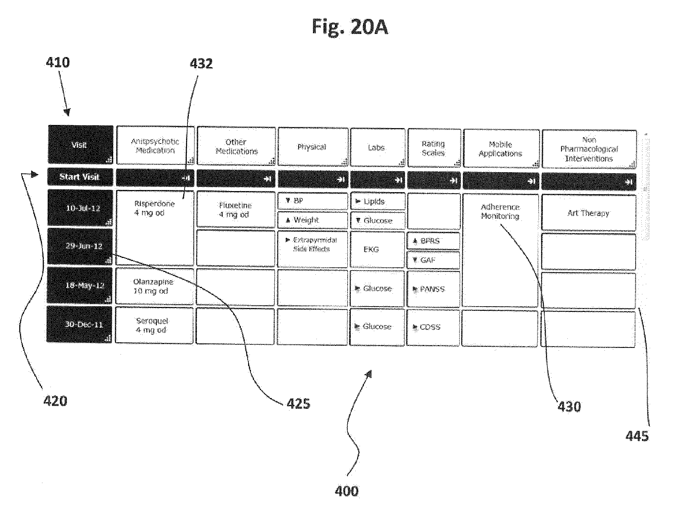

Fig. 20A is a partial representation of a timeline for use in the present invention;

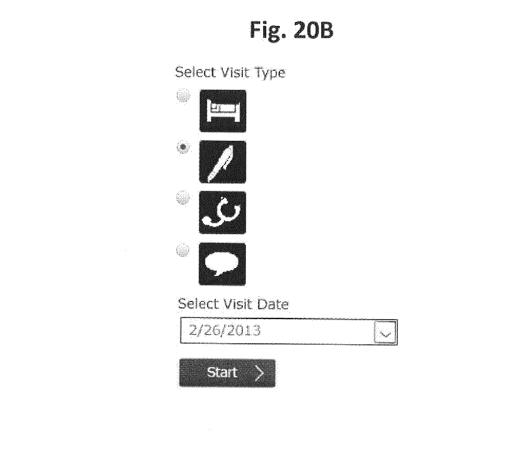

Fig. 20B is a partial representation of a visit selection display for use in the present invention;

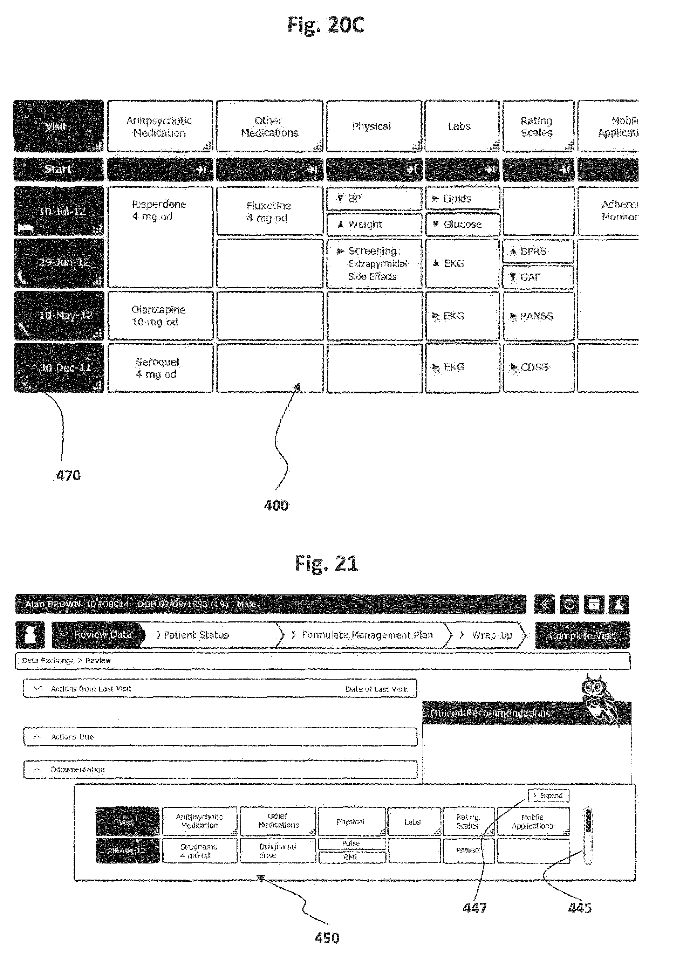

Fig. 20C is a partial representation of another timeline for use in the present invention;

Fig. 21 is a partial representation of a display screen with a minimised timeline for use in the present invention;

Fig. 22 is a schematic diagram of an advisory information system for use in the present invention;

Fig. 23 is an exemplary extract of a table of influences for use in the present invention;

Fig. 24 is an exemplary extract of an individual side effect table for use in the present invention; and

Fig. 25 is an exemplary extract of a drug side effects weighting table for use in the present invention.

**Detailed Description**

*General Architecture*

The present invention provides a personalised medicine system, which may be a specialised electronic device having only the function of the personalised medicine system. More usually the personalised medicine system of the present invention is an existing electronic device, such as a computer, adapted to have the functionality required by the present invention. Moreover, the personalised medicine system of the present invention may be distributed between several electronic devices/computers which are connected by one or more of a wired LAN, a wireless LAN, a WAN and the Internet.

Where the personalised medicine system is embodied as single electronic device it may be an existing mobile communications device such as a smart phone (for example iPhone™ or Android™ mobile/cell phone) or a tablet computer (for example iPad™ or Samsung Galaxy™ tablet). Such a mobile communications device can be adapted to have the functionality of a reminder device according to the present invention by downloading an application or widget to the mobile communications device. In some embodiments, it may interact with other devices to provide the functionality. In others, the mobile communications device may operate in a stand alone fashion.

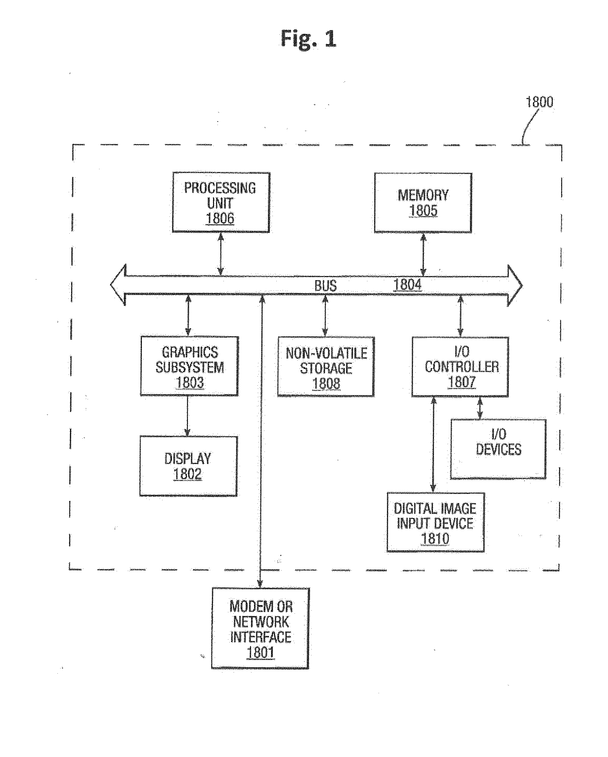

Fig. 1 illustrates an exemplary computer architecture 1800 by which a device embodying or forming part of the personalised medicine system according to the present invention may be implemented. Computer architecture 1800 may be or form part of a desktop computer or a laptop computer, a server, a mobile communications device or any similar computer device.

The computer architecture 1800 may interface to external devices such as another computer, the cloud or Internet, through a modem or network interface 1801, such as an analogue modem, ISDN modem, cable modem, token ring interface, or satellite transmission interface. The network interface 1801 may also be a standard communications module adapted to communicate with a standard mobile communications network, such as 2G, GSM, GPRS, EDGE, 3G, UTMS, CDMA 2000, HDSPA, LTE, 4G, etc networks; a Bluetooth radio; a Wi-Fi radio; or a combination of any two or more of the foregoing.

As shown in Fig. 1, the computer architecture 1800 includes a processing unit 1806, which may be a conventional microprocessor, such as commonly provided by Intel, ARM or AMD, which are known to one of ordinary skill in the computer art. System memory 1805 is coupled to the processing unit 1806 by a system bus 1804. System memory 1805 may be a DRAM, RAM, static RAM (SRAM) or any combination thereof. Bus 1804 couples processing unit 1806 to system memory 1805, to non-volatile storage 1808, to graphics subsystem 1803 and to input/output (I/O) controller 1807. Graphics subsystem 1803 controls a display device 1802, such as a liquid crystal display device, which may be a touchscreen device and/or may be part of the graphics subsystem 1803. The other I/O devices may include one or more of a keyboard, disk drives, printers, a mouse, a speaker, a camera 1810 and the like as known to one of ordinary skill in the computer art.

In one embodiment, the personalised medicine system device 1 is implemented using an application or a widget loaded onto a mobile communications device (such as a smart phone or tablet computer) having a touch screen display device 1802 and a camera 1810 and a speaker (not shown) as I/O devices, and optionally a microphone (not shown) as a further input device. The application causes the device 1 to store predetermined data in the non-volatile storage 1808, which is used to provide the required functionality.

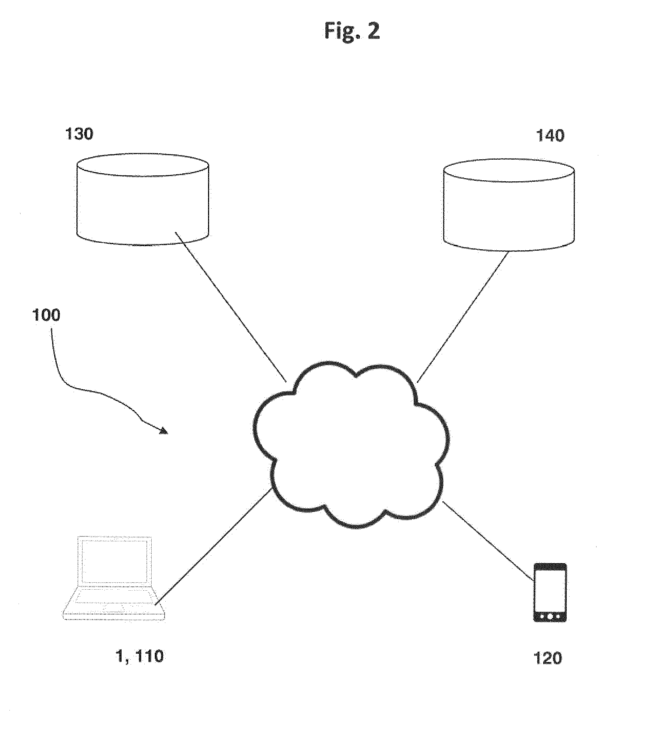

Fig. 2 shows a personalised medicine system 100 comprising a clinician computer 1, 110, a patient smartphone 120, and external servers/computers 130, 140 all of which are connected by one or more of a LAN, a WAN and the Internet. As will be discussed in detail below, the personalised medicine system stores various data, such as workflow information, page information, patient information, slider placement calculation information, side effect severity calculation information, knowledge base information and so forth. Such information may all be stored only on the clinician computer 1, embodying a personalised medicine system of the present invention by itself, or distributed between the clinician computer 110 and one or more networked or remote servers/computers 130, 140, which together embody the personalised medicine system 100 of the present invention. Likewise, processing operations required to provide the functionality of the personalised medicine system 100 may be carried out by the clinician computer 1 alone or may be distributed between the clinician computer 110 and the servers/computers 130, 140. Accordingly, where this specification discusses a particular device such as device 1, 110 storing particular information or carrying out a particular function, it should be understood that in different embodiments the storage and/or functions may be carried out by another device 130, 140 as well or instead.

The patient smartphone, tablet computer or other patient device 120 may provide information to the system 100, such as a record of patient adherence to a treatment regimen, as will be discussed in more detail below.

In some embodiments, multiple clinicians' computers 110 and/or patient devices 120 may be included in the system of the present invention.

*General Operation*

The personalised medicine system 1, 100 operates to guide a clinician through a series of interviews with a patient to assist the clinician to gather information about the patient and to decide on appropriate treatment. Thus, the personalised medicine system 1, 100 acts as a clinical decision support system (CDSS) and provides a personalised patient pathway to tailor diagnosis and treatment of an illness or other medical condition to specific patients. In the following description, the personalised medicine system 1, 100 will be described with reference to schizophrenia as an example of an illness/medical condition to which the personalised medicine system is tailored. However, it should be understood that the personalised medicine system 1, 100 can be tailored to other illnesses/medical conditions such as rheumatoid arthritis and cancer, as well as being applied to illnesses/conditions in general.

In order to carry out this functionality, the personalised medicine system 1, 100 stores a plurality of pages for displaying to a user and one or more workflows, each of the pages being associated with a step or node in a workflow. Each node in the workflow has one, two more pages associated with it.

In the present embodiment, the personalised medicine system 1, 100 stores three workflows, Workflow 1 for a first consultation with a patient, where the patient has not previously reported a contact with a psychiatrist or other mental health professional; Workflow 2 for a first consultation where the patient has previously reported a contact with another psychiatrist or mental health professional; and Workflow 3 for a subsequent consultation using the personalised medicine system 1, 100.

When a user (who will generally be a psychiatrist or other clinician) first starts the personalised medicine system 1, 100, for example by clicking an icon on a desktop or tapping an icon on the touch screen display device 1802 of a tablet computer, or navigating their browser to a specific address on a PC or mobile device, the personalised medicine system 1, 100 first displays a welcome screen (not shown). Here the user is able to select an existing patient previously stored in the system 1, 100 or create a new patient. If the user creates a new patient in the system, he is given the option to select whether or not this is patient's first psychiatric contact. Depending on the choices the user makes at this stage, one of the three respective workflows is selected. Each of the three workflows includes a plurality of stages, comprising one or more steps; one or more nodes associated with each step; and one or more pages associated with each node. One or more knowledge bases may be used to populate various pages. Commencement of a workflow starts a "visit", representing a consultation between the clinician and the patient during which information is reviewed and or recorded in the personalised medicine system 1, 100.

As will be discussed in more detail below, users can also access previous visits and use the workflow to move around the visit to review and/or adjust previously recorded information, as well as to add new information.

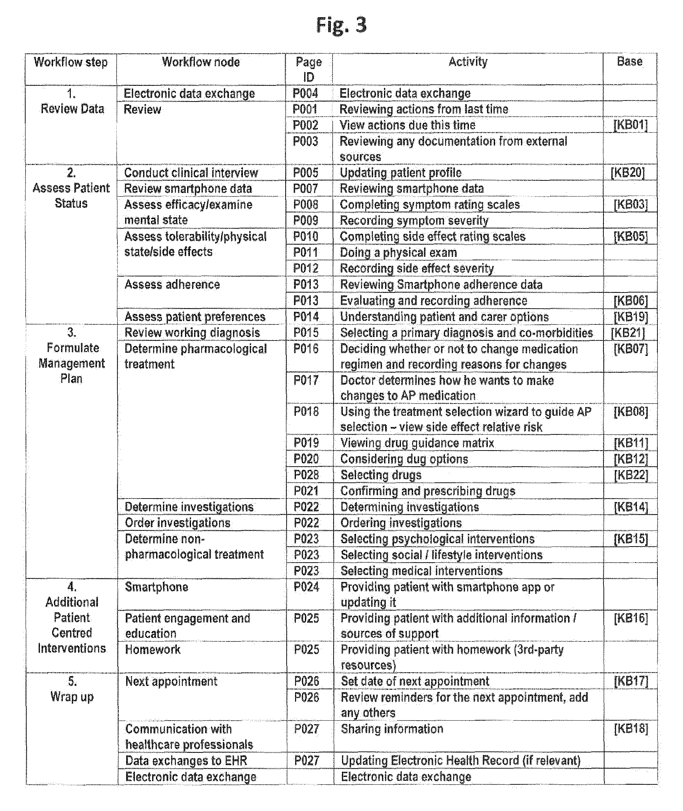

Fig. 3 shows in tabular form Workflow 3 provided for a subsequent consultation by way of example. The other two workflows are similar but changed to take account of their different purposes. The present invention will be described by way of example with reference to the workflow shown in Fig. 3 with relevant changes to the other workflows explained below.

The first workflow step in Fig. 3 for a new consultation with an existing patient is to Review Data and includes two nodes - Electronic Data Exchange and Review. The Electronic Data Exchange node is associated with page ID P004 (not shown). This allows the user to cause the personalised medicine system 1, 100 to interact with other healthcare IT technologies in the clinic/hospital/health authority to carry out automatic data exchange, for example to import patient details stored in electronic health/medical records (EHRs or EMRs) elsewhere, to view previously prescribed and to prescribe drugs, to order lab tests and obtain the results of previously ordered tests. This node may also allow the user to manually enter data, particularly where this is the first psychiatric contact in Workflow 1.

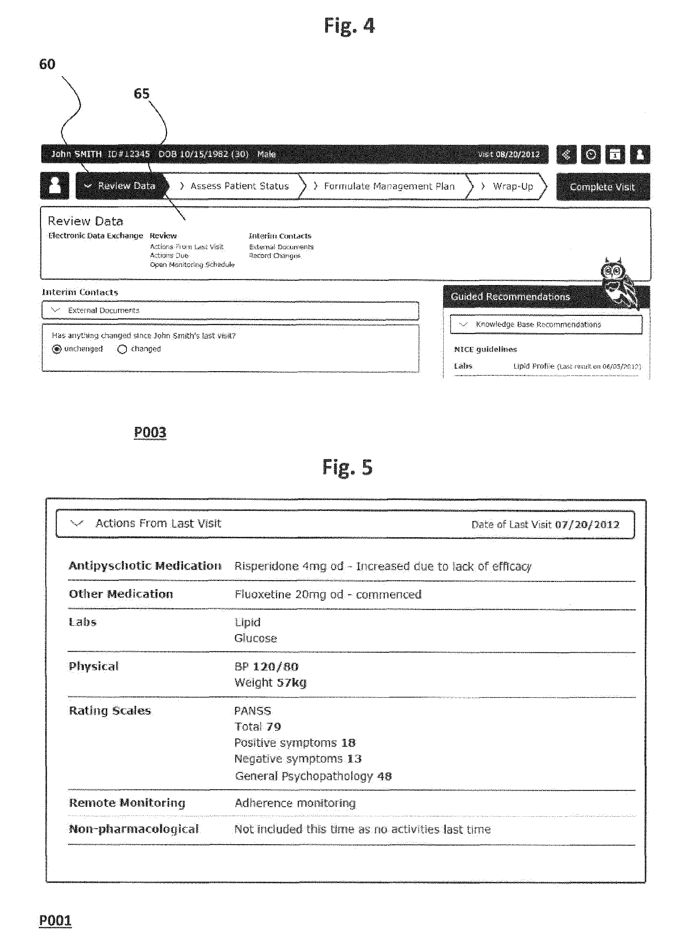

The workflow then moves to the Review node, causing it to display an associated screen. An extract of the screen heading is shown in Fig. 4, which includes the workflow steps in a main heading area 60 and the nodes for the selected step in a sub-heading area 65. The sub-heading area 65 also displays for each node a list of the related activities for which the associated pages are provided. Users can navigate through the workflow by clicking on the respective steps to select them. Once selected, a step is highlighted and a screen sub-heading shows each of the nodes for that step and each of the pages associated with the node. Thus, the user is also able to navigate between nodes and pages by clicking on them to select them. This display structure allows the user to navigate through the workflow in an easy way. Preferably, however, NEXT and PREVIOUS buttons and/or arrows are displayed at the bottom of the screen or elsewhere to allow the user to navigate through a workflow sequentially during a consultation.

In Fig. 4 it can be seen that the user is in the Review Data step of the workflow and is provided with options to enter the Electronic Data Exchange node (discussed above) and the Review node. Within the Review node, the user is able to access pages for reviewing actions from the last visit [page ID P001], viewing actions that are due to be taken this visit [P002] and reviewing documentation from external sources [P003]. In fact, in Fig. 4 the implementation is slightly different from the workflow in Fig. 3 and the option to review documentation from external sources is provided in a separate Interim Contacts node, where it is envisaged that the clinician will view documents created and information changed during an interim contact of the patient with another medical professional since the last consultation. Such interim contacts may occur, for example, because the patient has been admitted to hospital or has obtained psychiatric treatment from another person while on an extended stay at another location. The workflow may be as shown in Fig. 3 or illustrated in Fig. 4.

If the user selects the Review Actions from Last Visit option, for example by clicking on the corresponding wording using a mouse, then page P001 shown in Fig. 5 will be inserted in the screen shown by the display device 1802. Although not shown in Fig. 5, heading area 60 and sub-heading area 65 would also be displayed. Page P001 shows which actions took place as a result of the last visit. Thus, Fig. 5 shows that in the last visit the patient was prescribed risperidone as an antipsychotic medication, fluoxetine as other medication, lipid and glucose labs were ordered, blood pressure and weight were measured, various rating scales were used to assess the patient's symptoms and side effects suffered by the patient, and so forth.

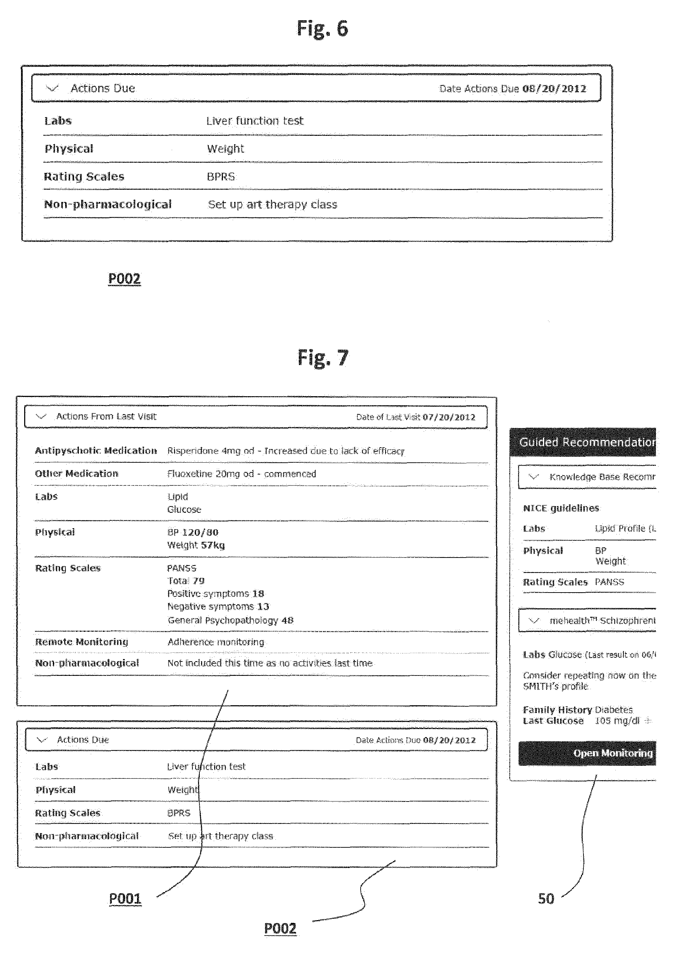

Fig. 6 shows the page P002 by way of further example, which provides the user with a list of actions that should be taken during this consultation.

As illustrated in Fig. 7, in some cases the personalised medicine system 1, 100 may display two or more pages at the same time on the display device 1802. In particular, Fig. 7 shows the concurrent display of pages P001 (actions from last visit) and P002 (actions due). Moreover, as shown in Fig. 7, in addition to displaying the selected pages P001, P002 and P003, the personalised medicine system 1, 100 may simultaneously display guided recommendations for the clinician in guidelines area 50. As before, heading area 60 and sub-heading area 65 would also be displayed simultaneously.

In workflow 3, following completion of the Review Data step, the workflow moves to the Assess Patient Status step and in particular the first node of conducting a clinical interview. In workflows 1 and 2, since this is the first time the patient has been entered in the personalised medicine system 1, 100, there can be no review of existing data and no due actions will have been set. Accordingly, workflows 1 and 2 omit the Review node of the Review Data step and, following electronic data exchange, move directly to the Assess Patient Status step of the workflow.

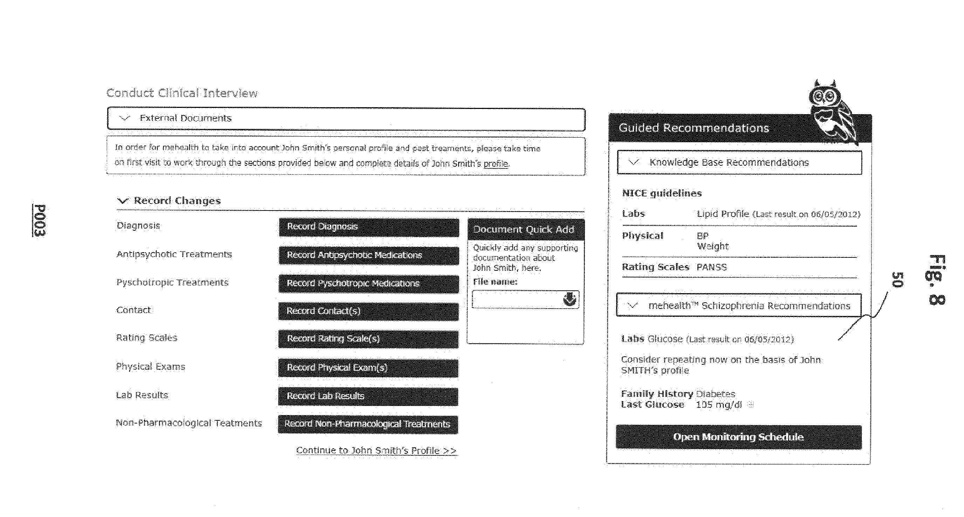

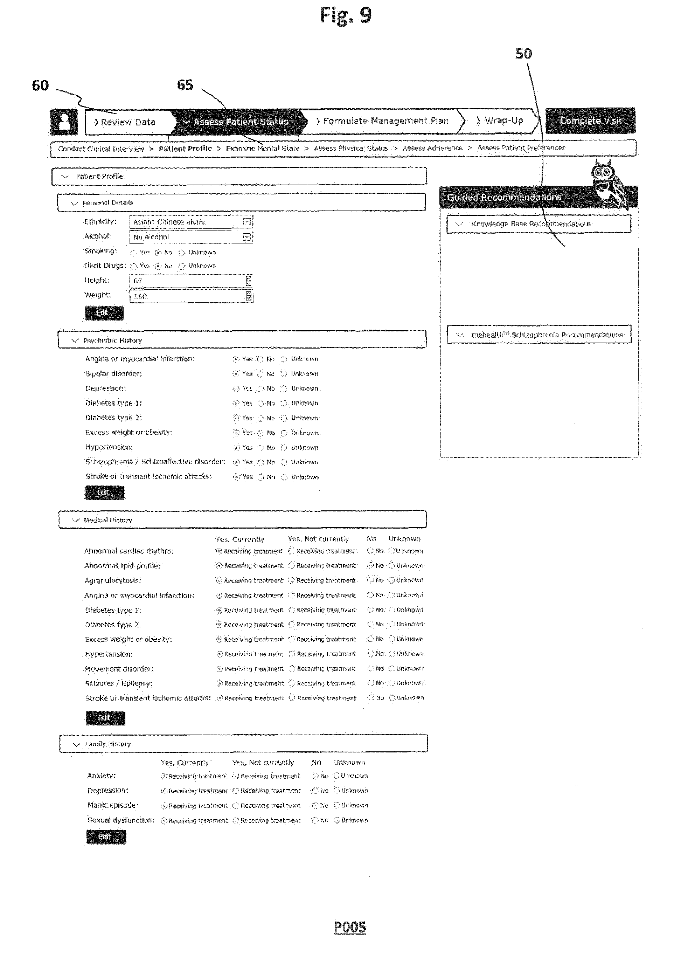

In the Assess Patient Status step of workflows 1 and 2, the first node of conducting a clinical interview is associated with pages P003 (discussed above in respect of workflow 3) and P005. In workflow 3, the conduct clinical interview node is associated only with page P005. Fig. 8 shows an example of page P003 in workflows 1 and 2 together with guidelines area 50. Fig. 9 shows an example of page P005 with guidelines area 50. In workflows 1 and 2, the clinician records the patient profile and clinical history from any available source, including the patient, by systematically working through the prompts in page P003. As shown in Fig. 8, these may include an existing (if any) diagnosis, antipsychotic treatments, other psychotropic treatments, contacts, rating scales, physical examinations, results of laboratory tests, non-pharmacological treatments and so on. Similarly, the clinician completes the patient profile in workflows 1 and 2 or updates the previously-completed patient profile in workflow 3 by following the prompts shown in Fig. 9.

Workflows 1 and 2 then move onto the "examine mental state" node, whereas workflow 3 first allows the clinician to review smartphone data in the "review smartphone" node in conjunction with page P007. In particular, during the (preferably first) visit, the patient's smartphone 120 is updated with an application that allows him to record when he takes his medications and how he is feeling at a given time. The data that has subsequently been input by the patient is then transferred from the smartphone or other patient device 120, for example at predetermined times or during a consultation, onto the clinician's computer 1, 110, and is used to populate page P007 for review by the clinician during the consultation.

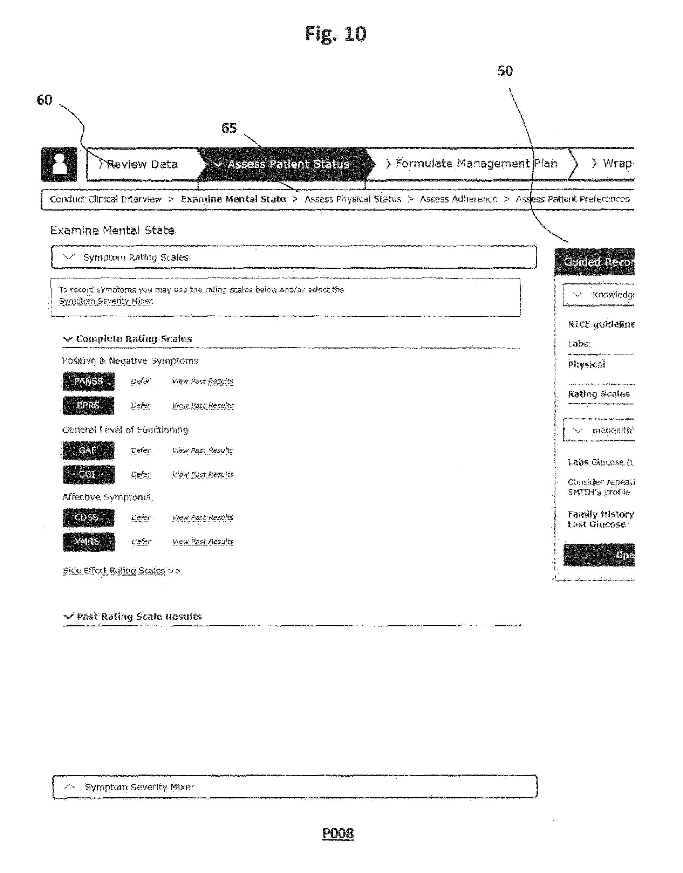

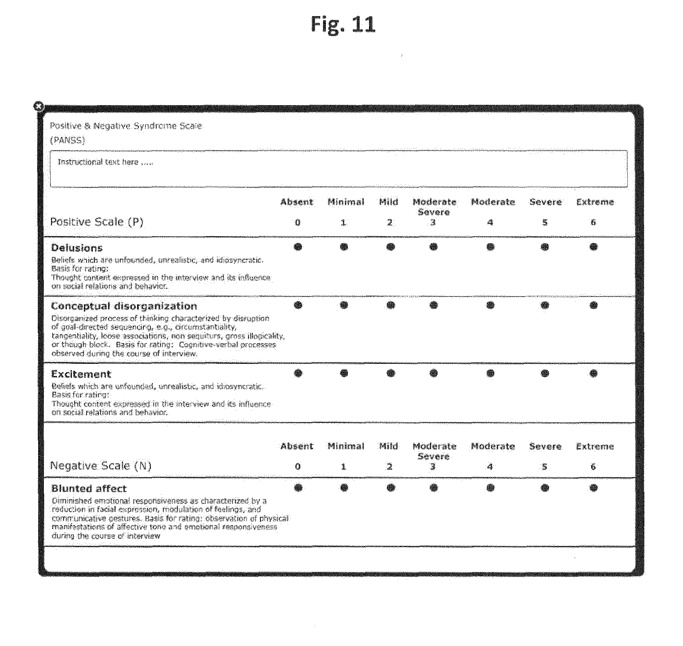

In the "examine mental state" node (which corresponds to the "assess efficacy" node in workflow 3), the user is presented with the option of completing one or more well-established rating scales for assessing the mental state of people suffering psychotic disorders such as schizophrenia. Thus, Fig. 10 shows the display of the screen header 60, 65 highlighting the Assess Patient Status step in the workflow and the "examine mental state" node together with the guidelines area 50. The exemplary symptoms ratings scales shown in Fig. 10 are the positive and negative syndrome scale (PANSS), brief psychiatric rating scale (BPRS), global assessment of functioning (GAF) scale, clinical global impression (CGI) scale, Calgary depression scale for schizophrenia (CDSS) and young mania rating scale (YMRS). However, any number of rating scales may be provided and the user can select any one of the available scales by clicking the corresponding icon to cause a pop-window to open, for example as shown in Fig. 11. The user can then complete the rating scale.

For example, the PANSS is a medical scale used for measuring symptom severity of patients with schizophrenia. To assess a patient using PANSS, an approximately 45-minute clinical interview is conducted. The patient is rated from 1 to 7 on 30 different symptoms based on the interview as well as reports of family members or primary care hospital workers. The symptoms are classified into a positive scale, a negative scale and a general psychopathology scale. The positive sub-scale includes 7 items, minimum score = 7, maximum score = 49 (delusions, conceptual disorganization, hallucinations, hyperactivity, grandiosity, suspiciousness/persecution and hostility); the negative sub-scale includes 7 items, minimum score = 7, maximum score = 49 (blunted affect, emotional withdrawal, poor rapport, passive/apathetic social withdrawal, difficulty in abstract thinking, lack of spontaneity and flow of conversation, and stereotyped thinking) and the general psychopathology sub-scale includes 16 items, minimum score = 16, maximum score = 112 (somatic concern, anxiety, guilt feelings, tension, mannerisms and posturing, depression, motor retardation, uncooperativeness, unusual thought content, disorientation, poor attention, lack of judgment and insight, disturbance of volition, poor impulse control, preoccupation, and active social avoidance).

The other scales operate in a similar manner but include different numbers of symptoms and different scales. For example, the GAF scale is simply a single number selected by the clinician based on his assessment of the patient's overall functioning, based on guidance the scale provides for different number ranges within the scale. Although the different scales may test for different things, generally there is some overlap between scales and different scales may test for (some of) the same things in different ways, for example with different questions and/or different numbers of questions. Other scales may also assess the patient's well-being in different non-clinical domains, for example, quality of life.

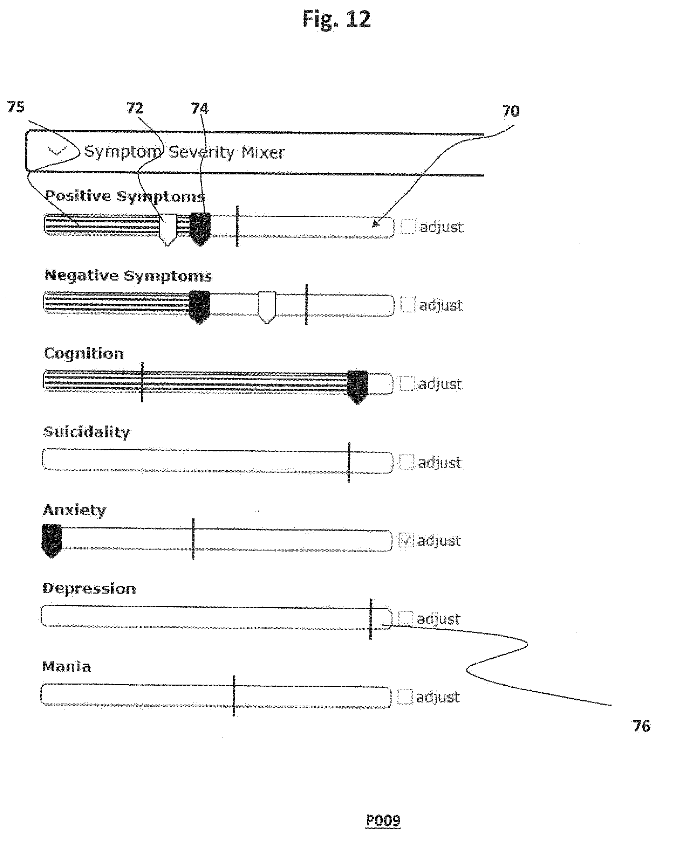

Based on the completion of one or more scales (or none) by the clinician, the personalised medicine system 1, 100 calculates a symptom severity value for each of a plurality of predetermined symptoms and displays the results of the calculations on a graphical scale for each of the symptoms on page P009, as illustrated in Fig. 12. In particular, page P009 shows a graphical representation of linear scale 70 for each of positive symptoms, negative symptoms, cognition, suicidality, anxiety, depression, and mania. Although not illustrated, other scales could also be provided, for example quality of life, and general level of functioning, although this is not an exhaustive list. These graphical representations may be termed symptom (severity) sliders 70 or mixers. In addition to populating the sliders 70 based on the clinician's data input to one or more rating scales in page P008, the user is able to enter a value on the scale based on his own impressions or additional information gained during the visit. Thus, in Fig. 12 shows for each slider 70 a light icon 72 representing a normalised value calculated by the personalised medicine system 1, 100 based on completion of one or more rating scales; a dark icon 74 representing a value enter by the clinician; a fill 75 from 0 up to the dark icon 74 to indicate clearly that the most important value is the clinician updated value; and a line 76 representing the previous value set during the previous visit (which corresponds to the previously set clinician icon 74 or, if the clinician did not input this, the previously calculated normalised value based on completion of one or more rating scales).

Preferably, the user enters a value manually by clicking on the selected slider and dragging an icon along the scale to the appropriate position. If a value has already been calculated by the personalised medicine system 1, 100 or a previous value has been stored, this will be displayed on the slider and the user will be able to use this information together with his experience to position the manually input slider. Alternatively the user may be able to enter a value in box (preferably from 0-10 or 0-100) and this will position the manually input slider on the graphical scale.

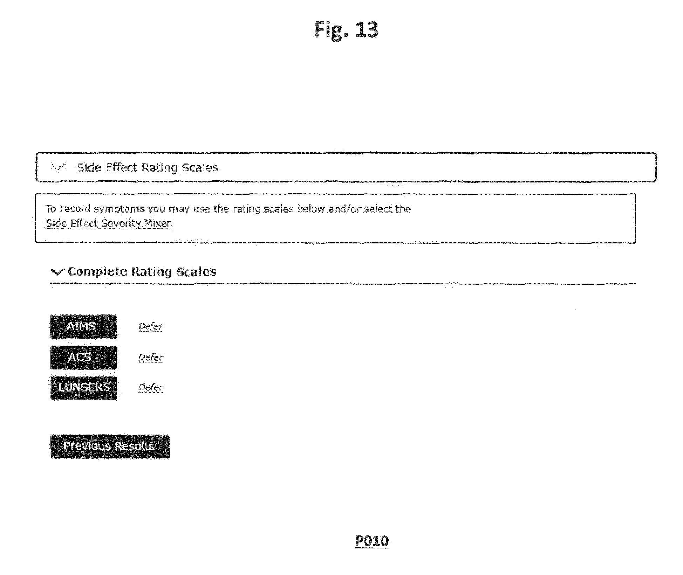

Following completion of the "examine mental state"/"assess efficacy" node, the workflow then moves on to the "assess physical status/side effects"/"assess tolerability" node. This node is associated with page P010, shown in Fig. 13. In this case, the user is given the option to complete one or more predetermined ratings scales to assess side effects of medications experienced by the patient. In the example, the scales are the abnormal involuntary movement scale (AIMS), the antipsychotic side-effect checklist (ASC) and the Liverpool University neuroleptic side effect rating scale (LUNSERS). Again, any number of other rating scales may be provided and again clicking one of the icons causes a pop-up window to open to guide the user through completion of the respective scale.

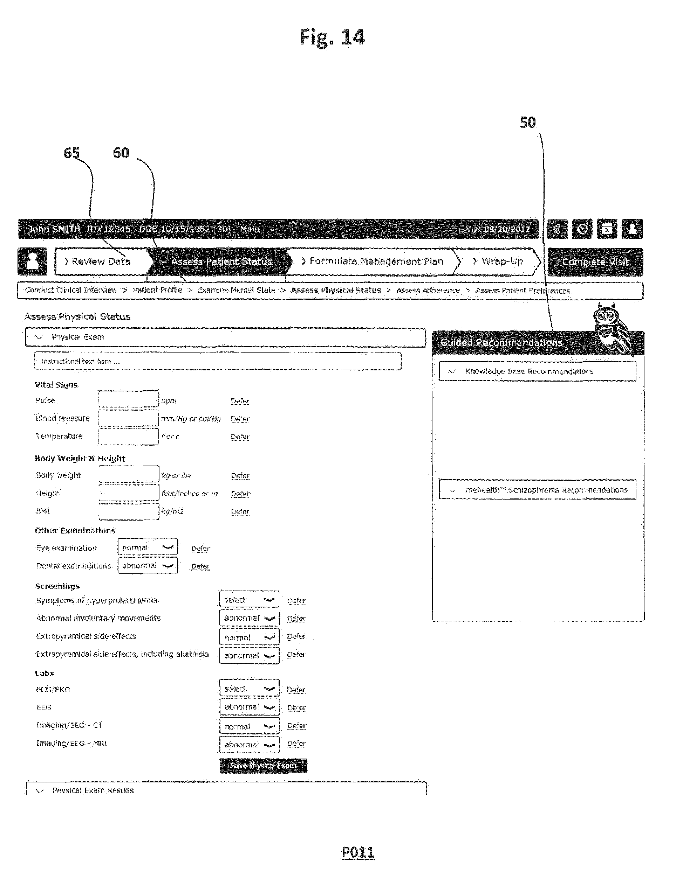

The next page associated with the node is the physical exam page P011. Fig. 14 shows an example of page P011 together with the relevant screen heading 60 and sub-heading 65, as well as the guidelines area 50. As with the other pages, alternative examples are possible. In any event, the user systematically follows the options presented on the page to enter the respective patient details such as height, weight, blood pressure and so forth.

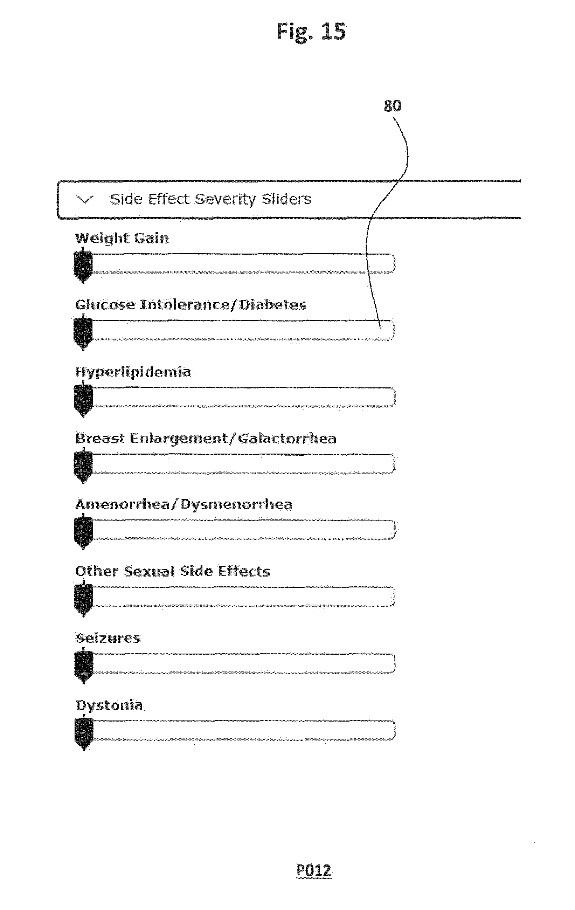

Following completion of pages P010 and P011, the personalised medicine system 1, 100 proceeds to display page P012, shown in Fig. 15, showing side effect (severity) sliders 80. These are populated based upon the completed side effect rating scales and, preferably, the input physical examination data. Similarly to the symptom severity sliders discussed above, the side effect severity sliders include a light icon representing a normalised value calculated by the personalised medicine system 1, 100; a dark icon representing a value manually entered by the clinician; a fill from zero up to the dark icon to indicate clearly that the most important value is the clinician updated value; and a line through the scale representing the previous value set during the previous visit (which corresponds to the previously set clinician icon or, if the clinician did not input this, the previously calculated normalised value based on completion of one or more side effect rating scales).

The user manually enters a value in a side effect severity slider in the same way as discussed above in respect of the symptom severity sliders.

The workflow then moves on to the "assess adherence" node to allow the user to assess how well the patient has adhered to previous or on-going treatment, for example by reviewing data from the patient's smartphone 120 and by checking boxes and entering other information as guided by the corresponding page(s). This node may be omitted or significantly altered in workflows 1 and 2, for example, if the patient is not currently prescribed any medication.

Finally in the Assess Patient Status step, the workflow moves on to the "assess patient preferences" node, where the clinician and patient decide whether to continue with existing medication or to change medication. If a decision to change medication is taken, a pop-up window may open giving the user the option to enter reasons for the change, such as partial efficacy, lack of efficacy or poor tolerability. Again, this node may be omitted or significantly altered in workflows 1 and 2.

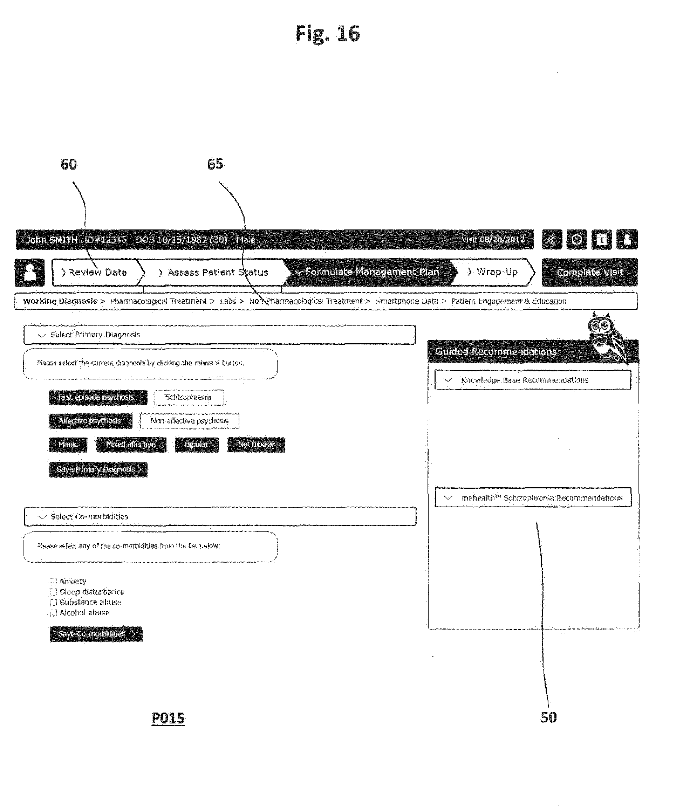

The workflow then moves on to the Formulate Management Plan step. In the first node, "review working diagnosis", page P015 is displayed, as shown in Fig. 16, allowing the user to select or confirm a primary diagnosis and co-morbidities. Preferably, the display of page P015 may change as the primary diagnosis is entered. For example, it may first offer the option of selecting first episode psychosis (FEP) or schizophrenia as the primary diagnosis. If FEP is selected, the page may then offer the selection of affective psychosis or non-affective psychosis. If affective psychosis is selected, the page may then offer the selection of manic, mixed effective, bipolar and non-bipolar. The page also allows the selection of co-morbidities, for example, anxiety, sleep disturbance, drug abuse and alcohol abuse.

In workflows 1 and 2, a "select primary diagnosis" or "determine working diagnosis" node substitute for the "review working diagnosis" node.

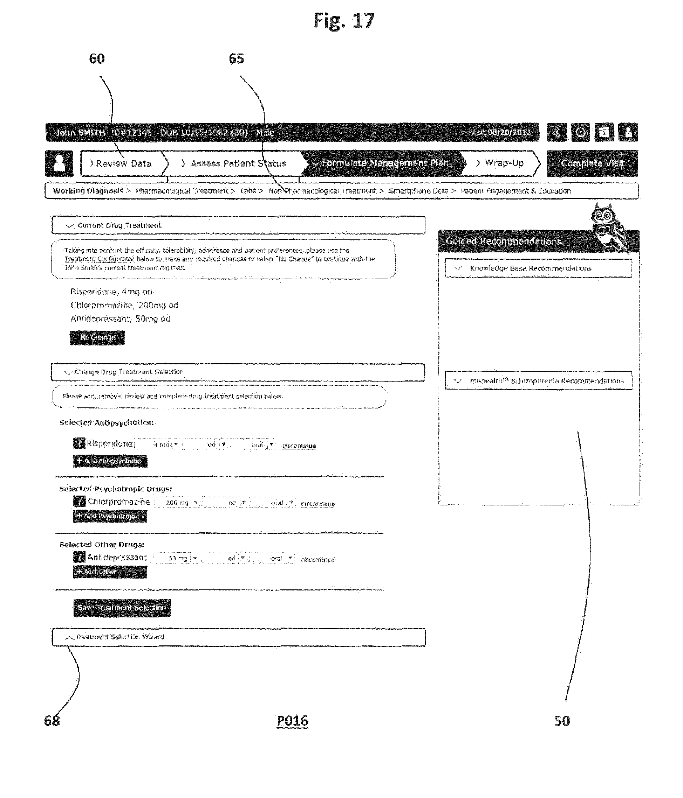

The workflow then moves on to the "determine pharmacological treatment" node, involving the sequential display of pages P016, P017, P018, P019, P020, P028 and P021. Page P016 is shown in Fig. 17 together with heading 60, sub-heading 65 and guidelines area 50. In page P016 the user is shown the current drug treatment regimen and the ability to change the regimen. If the user decides to make a change, pop-up window P017 (not shown) opens requesting the user to select one or more reasons for the decision. As shown in Fig. 17, the user may select one or more new drugs in page P016.

If the user decides not to make any changes, the workflow proceeds to show page P020. In the screen shown on display device 1802, the personalised medicine system 1, 100 provides an option of using an antipsychotic (AP) drug selection wizard or routine provided by the personalised medicine system 1, 100 (see section 68 in Fig. 17) at the same time as showing page P016. If the user decides not to use the wizard, the workflow accepts the changes made in page P016. Otherwise, the workflow moves to page P018.

It will be appreciated that instead of allowing the user to make changes to the drug selection in page P016, an intermediate page may be provided to allow the user either to use the personalised medicine system 1, 100 wizard or to manually select the AP drugs on a new page, which would be similar to the "change drug treatment selection" part shown at the bottom of page P016.

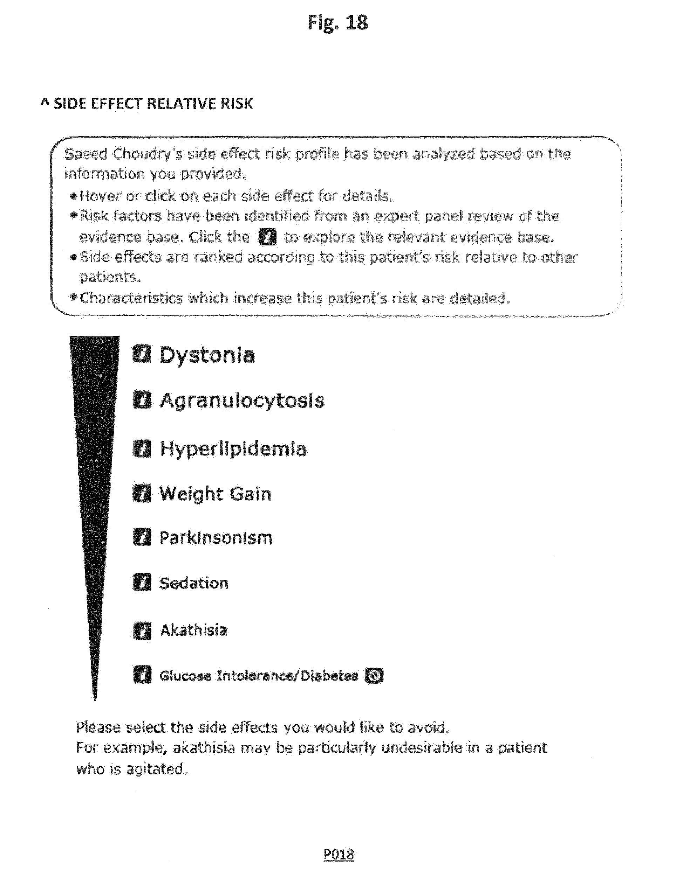

If the user decides to use the wizard, the personalised medicine system 1, 100 may first present, for a plurality of potential side effects, any characteristics which the patient possesses which increase the likelihood of experiencing the side effect. The strength of influence on the side effect of each specific characteristic is presented. The personalised medicine system 1, 100 then calculates, for a plurality of potential side effects, a risk that the patient will experience the side effect relative to the general population. The side effects are then shown in ranked order in page P018, as illustrated in Fig. 18. The manner in which the relative side effect risks and ranked order are determined will be discussed in more detail.

The user may optionally select one or more of the listed side effects which the clinician or patient wants to avoid. For example, a particular patient may particularly want to avoid weight gain or a clinician may recognise that akathisia is undesirable in a patient who is already agitated. A selected side effect may be shown in a different colour, size or font to the other side effects shown on the page.

The user may also hover over or click on each side effect for further details of the side effect, general factors which moderate the side effect, and specific factors extracted from the patient data used in the calculation. The user may also click on an information button next to each side effect to explore the evidence base for the side effect.

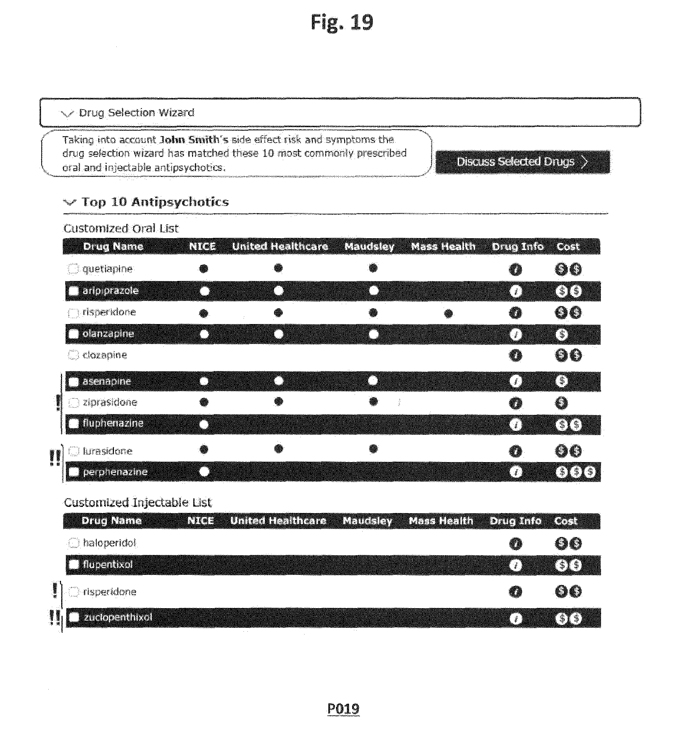

Depending on the working diagnosis selected in page P015, the personalised medicine system 1, 100 then displays in page P019 a list of AP drugs which could be used to treat the patient (see Fig. 19). Moreover, as shown in the figure, the personalised medicine system 1, 100 displays the list of selected AP drugs together with an indication for each drug of whether it is recommended/approved in respective predetermined clinical guidelines by placing a column for each guideline alongside the list of drugs and placing a dot or other marker in each column for each recommended/approved drug. In the example in Fig. 19, the predetermined guidelines are those published by the UK National Institute for Health and Clinical Excellence (NICE), UnitedHealthcare in the US, the Maudsley psychiatric hospital in the UK, and MassHealth in the US. Naturally, these guidelines are shown by way of illustration only and any other guidelines could be chosen as well or instead.

A column is also provided to allow the user to click on an icon for each drug to obtain further information about the drug, for example from one or more of the guidelines, the manufacturer or other sources. Optionally, the user can click on a dot or other icon under the guideline columns to view information in the respective guideline about the respective drug.

A further cost column is provided showing the relative cost of each drug. Again, clicking on an icon in this column may show the user further information about the costs of the respective drug.

Page P019 further includes an indicator (shown on the left hand side in Fig. 19) of the relative risks of each of the displayed drugs to the particular patient, depending on the side effect relative risks calculated by the personalised medicine system 1, 100, a side effect selected to be avoided in page P018 and, optionally, other patient data. In particular, in the example in Fig. 19, ten drugs to be taken orally are displayed with a single exclamation mark (!) placed against drugs 6-8 in the list and two exclamation marks (!!) placed against drugs 9 and 10. Similarly, five drugs to be taken by injection are displayed with a single exclamation mark (!) placed against drug 4 in the list and two exclamation marks (!!) placed against drug 5. A single exclamation mark may be used, for example, to indicate that the drug should be used with some caution, as the patient's profile suggests that he may have an increased risk of certain adverse side effects. Two exclamation marks may be used to indicate that the drug should be used with extreme caution, as the patient's profile suggests that he is likely to have an increased risk of adverse side effects. Clicking on an icon adjacent the drug may provide further information about what the adverse side effects are and the relative risk of experiencing them. The number of exclamation marks also takes into account the views of the patient and doctor about which side effects would be most undesirable.

Accordingly, the drug table provides a wealth of information tailored to the specific patient to allow the clinician and the patient to discuss suitable AP drug treatment options and select an AP drug for treatment.

Irrespective of whether the pharmacological treatment has been changed, the personalised medicine system 1, 100 then shows page P020 (not shown) which is populated with information about the selected AP drug, allowing further discussion of the treatment options. Subsequently, page P028 (not shown) is displayed, allowing the user to select non-antipsychotic psychotropic medications in addition to the selected AP drug. Optionally, page P028 is only displayed the first time an AP drug has been selected/prescribed or if the AP drug selection has changed. The workflow then moves on to shown page P021 (not shown), in which the user confirms and prescribes the selected drug(s).

The workflow then proceeds to the "determine investigations" node to determine which investigations should be carried out on the patient based on the prescribed drug(s), the patient's status, and other information (such as the evidence base and the guideline recommendations, which are stored in a knowledge base 260 discussed below), to obtain baseline values or follow-up evaluations of the patient's tolerability and physical health status; the "ordering investigations" node to order the determined investigations; the "selecting psychological interventions" node for selecting and ordering interventions such as family therapy, cognitive behavioural therapy (CBT) etc; the "selecting social/lifestyle interventions" node for selecting and ordering interventions such as smoking cessation support, gym referral, supported employment etc; and the "selecting medical interventions" node for selecting and ordering interventions such as dietary advice, alcohol/drugs therapy, diabetologist referral etc.

Finally, the workflow moves on to the Additional Patient Centred Interventions and Wrap-up steps of the workflow with their associated nodes and pages.

Accordingly, it will be appreciated that the personalised medicine system 1, 100 of the present embodiment provides a platform written specifically for clinicians and patients in the area of psychiatry, which addresses the unique needs of practitioners as they relate to patient tracking, clinical decision support, clinical references and guidelines, evaluation (treatment and outcome assessment), patient-facing support, and personalised remote self-monitoring. The personalised medicine system 1, 100 provides personalised best practice clinical management decisions by combining industry-recognised guidelines, a published evidence base, a clear workflow for the clinician to follow and the patient's unique characteristics. The present invention improves the care of individuals with chronic mental illness, where there is a large number of different treatments available, each with unpredictable efficacy and troublesome side effects. In addition to providing guidance on the selection of appropriate drugs for treatment of the patient, the personalised medicine system 1, 100 of the present invention also helps the doctor with making non-drug treatment decisions; obtaining, providing and reviewing patient information; monitoring, recording and presenting outcome etc.

Although the present embodiment relates to a personalised medicine system 1, 100 specifically adapted to the treatment of mental illness and, specifically, schizophrenia and other psychosis, it will be apparent that the personalised medicine system 1, 100 of the present invention can be adapted to a host of other illnesses, including especially those illnesses and conditions that require long term care and multiple consultations between patient and physician, such as various different forms of cancer, rheumatoid arthritis and so on.

However, the personalised medicine system 1, 100 of the present invention can also be adapted to provide a workflow for the general practitioner or trained nurse to follow in more general consultations in less specialised fields of practice.

In each case, the personalised medicine system 1, 100 of the present invention can provide point-of-care decision support for a wide spectrum of clinical decisions from diagnosis to chronic disease management. It accompanies the doctor and patient across the full continuum of care and throughout the illness, informing therapeutic decisions, monitoring outcomes and offering patient-focused interventions. In doing so, the personalised medicine system 1, 100 allows clinicians to apply the evidence-base to each individual patient and facilitates concordance of the treatment with selected guidelines, promoting consistently higher quality care. Preferably, it can be customised by healthcare providers and doctors to preferred guidelines or formularies.

Specific aspects of the personalised medicine system 1, 100 will now be described in more detail.

*Context Specific Information*

As previously discussed the personalised medicine system 1, 100 may display on screen, together with the page(s) associated with the relevant node of a step in the workflow, a guidelines area 50. In addition, as shown in Fig. 6, page P003 shows actions due in the current visit. Other such prompts may appear elsewhere in the workflow. Together, the guidelines area 50 and the other prompts due provide the user with context specific advisory information for the user to follow or review as desired. The provision of the context specific advisory information (guidelines area 50 and the actions due prompts) will now be discussed in more detail.

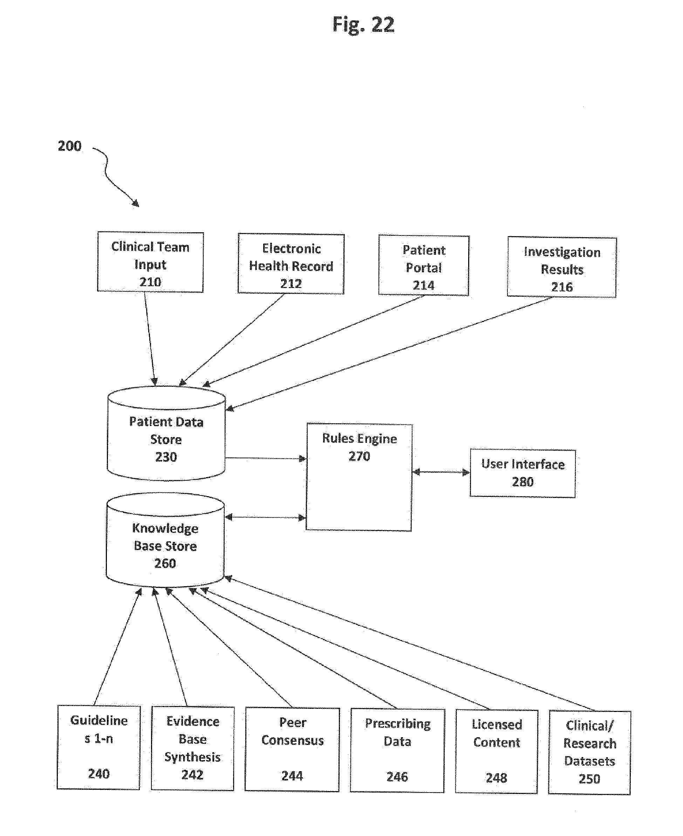

Fig. 22 is a schematic diagram of the advisory information sub-system 200 contributing to the advisory information functionality of the personalised medicine system 1, 100. Specifically, advisory information sub-system 200 comprises a patient data store 230 and a knowledge base store 260.

The patient data store 230 is populated for each patient with information about that patient derived from various sources, such as clinical team input 210; existing electronic health/medical records (EHR/EMR) for the patient 212; a patient portal 214 which receives inputs from the patient in clinic or via their mobile communications or other device 120 (eg smartphone or tablet) for example concerning their mood or taking of medications (including remote monitoring of the patient); and investigation results 216 for example from labs or results of personalised medicine tests, for example for genetic markers.

The knowledge base store 260 derives from inputs from various sources, including various different published clinical guidelines and treatment algorithms 240 (for example, from NICE, UnitedHealthcare and MassHealth); a synthesis of the evidence base 242 relating to the condition to which the personalised medicine system 1, 100 is tailored (for example, mental illness); peer consensus 244; predetermined prescribing data 246; licensed content (for example, a clinical textbook such as the Maudsley Prescribing Guidelines) 248; and clinical/research datasets 250 including de-identified patient data from the system. Known knowledge capture and data mining processes may be carried out on these sources to extract relevant information and to populate the knowledge base. Such processes may be carried out automatically using a computer program to parse and interrogate each knowledge source or by a human, or more usually by a combination of automatic and manual operations.

Naturally, the knowledge base store 260 stores a large number of such rules, together with the underlying data to display in the guidelines area 50 once a rule network was been parsed. Such rules may allow for or even require the input of various different items of patient data or other information to be successfully parsed.

These rules may be grouped into subsidiary knowledge bases, and the various pages may call upon the subsidiary knowledge bases to populate the guidelines area 50 or the advisory action section of the page. Thus, the table in Fig. 3 shows that page P002 in the "review" node of the Review Data step in workflow 3 calls upon subsidiary knowledge base KB01, page P005 in workflow 3 calls upon subsidiary knowledge base KB20, and so on.

It will be appreciated that the information stored and the processes performed may be distributed otherwise than as described above to achieve the same functionality. For example, rather than storing (all of) the advisory information, the knowledge base store 260 may store a link to the appropriate source 240, 242, 244, 246, 248, 250 and retrieve some or all of the required information on demand from the rules engine 270, or the rules engine 270 may retrieve the required information, possibly via the knowledge base store 260.

In addition, the rules engine 270 may receive feedback information from the user interface 280 to adapt the advisory information to be displayed. For example, where the knowledge base store 260 provides advisory information from a plurality of different guidelines (for example, one or more published guidelines and/or a proprietary guideline), the personalised medicine system 1, 100 may enable the user to select the guideline(s) on which advisory information should be based.

Moreover, as the sources of data 240-250 for the knowledge base store 260 evolve, so the knowledge base store 260 can evolve and be updated. This can occur automatically as the sources 240-250 change. Thus, if the rules lead to display of information from the prescribing data source(s) 246, and the data from the data source changes, this can be reflected in the data shown by the personalised medicine system 1, 100. For example, updates in the prescribing data 246 can be automatically imported into the knowledge base store 260, or the knowledge base store may provide a link to the same place, and the information at the link will change as the source evolves. Naturally, this applies to the other sources of data 240-250.

Moreover, the rules in the knowledge base store 260 may be updated automatically or manually too.

From the foregoing discussion of the general architecture, it will be apparent that each of the patient data store 230, the knowledge base store 260 and the rules engine 270 can be stored on the clinician's computer 1 in one embodiment. Alternatively, in other embodiments one or more of these may be stored on a remote server/computer 130, 140 and the clinician's computer 110 may access them via LAN, WAN and/or the Internet. Such an arrangement might be preferable, for example, where the clinician uses a tablet computer 110 during consultation to maximise interaction with the patient. In this case, the clinician's computer 110 embodying one aspect of the invention may store only the user interface 280 component as an application or widget and access the other components of the system by Wi-Fi.

In another embodiment, the user interface 280 and the rules engine 270 are stored on the clinician computer 110, the patient data store 230 is stored on a server 130 for a clinic, and the knowledge base store 260 is stored on a separate server 140. Multiple clinicians' computers 110, for example within a single hospital or health provider, may access patient data held by the patient data store 230 on the server 130. In addition, clinician's computers 110 for multiple clinics or health providers may access the knowledge base store 260 held on the server 140, which is maintained and updated by a third party.

It will be apparent that various other distributions of physical and software components of the invention are possible, and each is within the scope of the present invention.

*Side Effect Risk and Drug Mapping*

As described above, the personalised medicine system 1, 100 calculates, for a plurality of potential side effects, a risk that the patient will experience the side effect relative to the general population and displays the side effects in ranked order on page P018, as illustrated in Fig. 18. The personalised medicine system 1, 100 then displays the likely suitability for treatment of a patient of each of a list of drugs based at least in part on the calculated side effect risks. The manner in which the relative side effect risks and ranked order are determined, as well as the mapping of the side effect risks to the list of drugs will now be discussed in more detail.

As previously discussed, as the clinician works through the workflow during a consultation with a patient, he optionally carries out or populates patient information, for example side effect rating scales in page P010 (Fig. 13) and physical exams in page P011 (Fig. 17) and investigations in page P022. In addition, the clinician may change the side effect ratings by using the side effect sliders 80, illustrated in Fig. 15. The patient may also have access to an interface which allows him to record the severity of side effects and the distress which they cause.

Preferably, the personalised medicine system 1, 100 also offers the ability to include side effects previously experienced by the patient. These can be recorded by the clinician during a consultation based on the patient's previous clinical history before the personalised medicine system 1, 100 of the present invention was used for the patient, for example using workflow 2. In addition, the previously experienced side effects can be data recorded in previous consultations since the personalised medicine system 1, 100 was used for the patient. This previously recorded data can be the position of the sliders during a previous consultation or, if the patient stops treatment using a medication and the clinician records why. The data on the side effects previously experienced by the patient is used to position the vertical line on the slider 80 in Fig. 15.

This patient data is then used to calculate the relative risks and populate page P018 in Fig. 18. In particular, the present invention recognises that there are a number of risk factors (or moderators) that can affect each side effect, and uses these to assess an individual patient's risks of experiencing each side effect.

More specifically, the personalised medicine system 1, 100 stores, for example in the knowledge base 260, a table of influences used to calculate relative risks. The table of influences is multidimensional and includes a number of different layers (or conceptually, comprises a number of related tables).

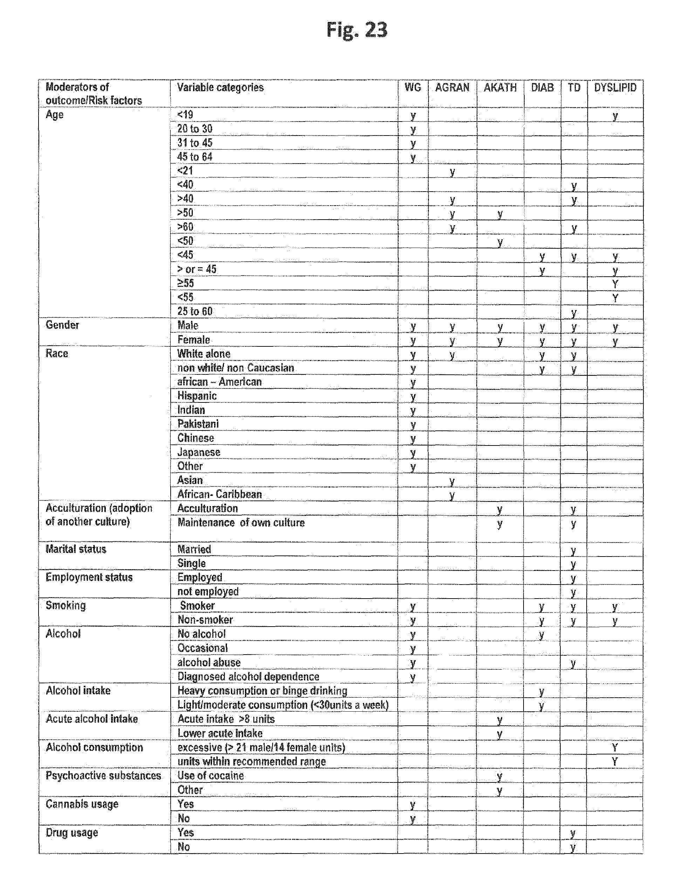

In a first layer, the table lists all of the different moderators that can affect the risk of experiencing a side effect together with (sub-) categories for each moderator and an indication of which categories affect which side effects. An exemplary extract of this layer of the table is shown in Fig. 23. The first column shows the moderators, the second shows each of the categories for each moderator and the subsequent columns show various side effects - in this case, weight gain (WG), agranulocytosis (AGRAN), akathisia (AKATH), diabetes (DIAB), tardive dyskinesia (TD), and dyslipidemia (DYSLIPID).

Each of the moderators relates to patient-specific information and it can be determined whether the patient falls into one of the categories of a moderator by extracting the relevant patient information stored in the patient data store 230. It can be seen that the categories within a moderator can overlap. Thus, in the example in Fig. 23, the age moderator includes the overlapping categories 31-45, <40 and 25-60. This is because the different side effects are affected differently by the different categories.

In the exemplary table in Fig. 23, only six side effects are shown. However, it is preferred that a more exhaustive list of side effects is used (even more exhaustive than the example shown in Fig. 15). Similarly, the table in Fig. 23 shows only a limited number of moderators, but in practice many more moderators will be used including not only moderators relating to personal information such as those shown in Fig. 23 but also family history moderators; medical physical history moderators (eg cardiovascular disease, obesity, liver disease); psychiatric history moderators; baseline investigation moderators (eg baseline lipid profile, baseline white blood count pre-clozapine); current investigation moderators; physical examination moderators (eg body mass index); current clinical moderators (eg current diagnosis, schizophrenia sub-type); past clinical moderators (eg time since first antipsychotic treatment); current medication moderators (eg dose); and so on.

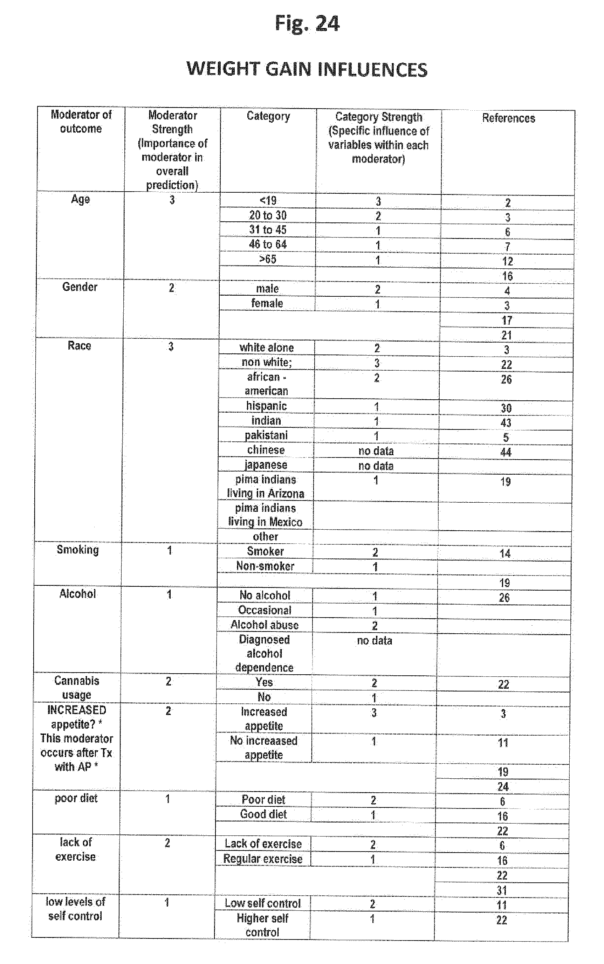

A separate layer of the table is provided for each side effect. Fig. 24 shows an extract of a layer provided for the side effect of weight gain. In the table, each moderator is assigned a moderator strength within a predetermined range (1-3 in the example) in accordance with the effect the moderator has on the likelihood of the patient experiencing the side effect relative to the other moderators. In addition, each category is assigned with a category strength within a predetermined range (1-3 in the example) in accordance with the effect the category has on the likelihood of the patient experiencing the side effect relative to the other categories for that moderator. Thus, in the table layer shown in Fig. 24, it can be seen that the moderator of age has a stronger effect that the moderator of gender on the risk of a patient experiencing the side effect of weight gain. Moreover, patients under 19 years old will be more likely to experience weight gain than a patient 20 to 30 years old, who in turn will be more likely to experience weight gain than older patients. Moreover, male patients are more likely to experience weight gain than female patients.

In order to determine a risk indicator for the patient experiencing a particular side effect, the personalised medicine system 1, 100 interrogates the table of influences and determines which factors moderate the risk of experiencing the side effect, and which categories of each relevant moderator the patient falls into. The personalised medicine system 1, 100 then combines the moderator strengths and category strengths to determine an overall risk indicator for that side effect.

Any suitable way of combining the moderator and category strengths may be used. Preferably, the moderator strength ($M_x$) of each relevant moderator is multiplied by the category strength ($C_x$) of the category in which the patient falls, and the results of the multiplications are summed to reach a total risk value V:

$$V = (M_1C_1 + M_2C_2 + M_3C_3 + ... + M_nC_n).$$

For example, referring to Fig. 24, a male under 19 years old would be 2.6 (13/5) times as likely to experience weight gain than a female over 65 years old, all other factors being equal. (3x3 + 2x2 = 13; 3x1 + 2x1 = 5).

Where n is the number of moderators for which information is available to classify the patient, Mmax is the maximum value for the moderator strength and Cmax is the maximum value for the category strength, a risk indicator I for a side effect can be calculated as:

$$I = V/ (n * Mmax * Cmax).$$

In the foregoing example, if only age and gender were known, the final risk indicator In for weight gain for the male under 19 years old would be 0.72 (13/(2*3*3)) for the female over 65 years old it would be 0.28 (5/(2*3*3)). Thus, by dividing the risk value V in this way, it is possible to obtain a value between 0 and 1 for each risk indicator.

For any one side effect, the category strengths need to be normalised across a predetermined scale. For example, in Fig. 24, all category strengths are between 1 and 3. This ensures that the values assigned to moderator strength properly reflect the strength of the moderator relative to the other moderators for the side effect, and the combined moderator and category strength results are not unduly influenced by the category strengths.

In addition, in order that risk indicators for different side effects can be usefully compared, if the range of category strengths differs between side effects, it is normalised for all side effects, for example to a scale of 1 to 9. This can be done in a number of ways but in the present embodiment normalisation is achieved by mapping category strengths for a side effect to a normalised scale in a predetermined way. Where the range of category strengths is from 1 to 9, no mapping is required. Where the range of category strengths is 1 or 2, 1 is mapped to 1 on the normalised scale and 2 is mapped to 9. Where the range of category strengths is 1 to 3, 1 is mapped to 1, 2 is mapped to 5 and 3 is mapped to 9 on the normalised scale. Where the range of category strengths is 1 to 4, 1 is mapped to 1, 2 is mapped to 3, 3 is mapped to 6 and 4 is mapped to 9. Where the range of category strengths is 1 to 5, 1 is mapped to 1, 2 is mapped to 3, 3 is mapped to 5 and 4 is mapped to 7 and 5 is mapped to 9, and so on. Moreover, the moderator strengths are selected to allow useful comparison between the risk indicators determined for each side effect.

It will be apparent that risk indicators can be determined for each side effect using different modes of combining moderator and category strengths or using different ways of ranking moderators/categories. For example, rather than multiplying moderator and category strengths, they can be added and the results for all moderators can be added. However, some normalisation is preferred so that risk indicators for the different side effects can be usefully compared with one another.

Once the personalised medicine system 1, 100 has calculated a risk indicator for each of the side effects, they are compared and the side effects are displayed in page P018 in order of, or with some indication of, the respective risk indicators. Thus, in Fig. 18 the relative risk of the patient experiencing dystonia is highest and so dystonia is displayed at the top in the largest font, and the relative risk of diabetes is lowest so diabetes is displayed at the bottom in the smallest font.

It should be noted that in the present embodiment, the risk indicator is an indication of the selected patient's risk relative to other patients. For example, if the patient is a 19 year old African American male with mid-range BMI, he is at a moderately higher risk than the general population of experiencing weight gain. However, he is at a lower risk than the general population of experiencing akathisia, and a higher risk than the general population of experiencing dystonia. However, although dystonia may be listed first and weight gain fourth, this does not mean he is more likely to get dystonia than weight gain. This is because weight gain is a more common side effect than dystonia.

If desired, each side effect can also be provided with a side effect strength S, which is multiplied by the risk indicator for the side effect to give a final side effect likelihood L (or risk indicator that has been modified):

$$L = S * I$$

In some embodiments, the user may be able switch between showing the side effects in order of L or I, or these may be shown concurrently, although this is not necessarily preferred.

The table in Fig. 24 also includes a column including references. When the user clicks on the "i" icon next to a side effect on page P018, he is taken to the references referred to in the table for each moderator and/or category and stored in the knowledge base store 260. Thus, if the user clicks for information relating to weight gain and the patient is under 19 years old, the user will be displayed the text of references 2 and 16 stored in the knowledge base store 260 or accessed from there but stored in peer consensus source 244, for example. The table may also include a table for comments to be displayed in the event the patient falls within a particular category used in the determination of the risk indicator.

In general existing practice, it is not possible for clinicians to take account of the large numbers of different factors that affect the likelihood of a patient undergoing treatment, particularly during a consultation, and clinicians rely on their own experience in assessing the likelihood of side effects when prescribing medications. However, the present invention provides the user with a side effect risk profile for the selected patient relative to the general patient population, which is a powerful tool in guiding the clinician during a clinical consultation. Moreover, the relative risk profile can take account of a vast array of sources of primary data to which the clinician may not have had access and will not be aware, and which may be counterintuitive to his own experience. Further, the relative risk profile is provided at an appropriate step in the consultation workflow, ensuring that it will be considered by the clinician at the appropriate time. Thus, the present invention significantly improves patient care and outcomes. The skilled addressee will appreciate that the side effect relative risk profile can be shown in any number of different ways, whilst still providing the foregoing advantages and benefits.

As noted above, the user may select side effects to be avoided. Depending on the working diagnosis selected in page P015 and, optionally, the side effect relative risks calculated by the personalised medicine system 1, 100 and the patient's own preferences the side effect(s) selected to be avoided in page P018 and other patient data, the personalised medicine system 1, 100 then displays in a particular order a list of suitable AP drugs for treatment of the patient (see Fig. 19) in page P019.

The drugs used to populate the list in page P019 will be selected based on rules and information stored in the knowledge base store 260, as described above. For example, the list of drugs may correspond to the 10 most popularly or commonly described drugs taken orally and the five most commonly prescribed drugs taken by injection in the knowledge base for patients having the same diagnosis and similar characteristics.

Page P019 further includes an indicator (shown on the left hand side in Fig. 19) of the relative risks of the displayed drugs to the particular patient. In particular, in the example in Fig. 19, a single exclamation mark (!) is placed against drugs 6-8 in the first list and two exclamation marks (!!) are placed against drugs 9 and 10. Similarly, a single exclamation mark (!) is placed against drug 4 in the second list and two exclamation marks (!!) are placed against drug 5.

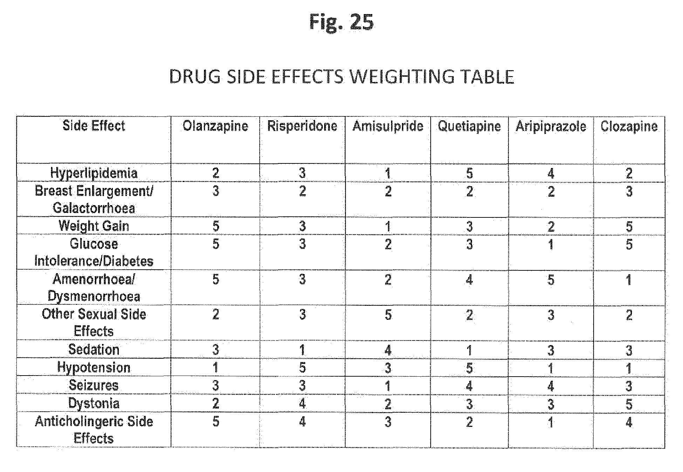

The determination of the order in which the drugs are displayed and of whether to place any exclamation marks against a drug may be carried out as follows. The knowledge base store 260 further holds a drug side effects weighting table, an exemplary extract of which is shown in Fig. 25. For each drug, a weighting is given for each of the possible side effects, reflecting the likelihood that the drug will cause the side effect. The weighting is given as a number within a predetermined range, in this example 1-5. Generally, the weightings reflect a likelihood of the drug causing patients to experience the side effect relative to the other drugs. However, in some embodiments the weighting may reflect an absolute likelihood of an average patient experiencing the side effect as a result of taking the drug.

In the present invention, the rules engine 270 retrieves the drug side effect weightings table from the knowledge base store 260. Then, for each drug in the table, the rules engine 270 multiplies the risk indicator I for each side effect previously calculated for the patient and multiplies it by the weighting W for the corresponding side effect. The results are then summed to obtain an overall drug weighting score Dw for the drug:

$$Dw = I_1 W_1 + I_2 W_2 + \ldots + I_n W_n$$

Thus, an overall drug weighting score Dw specific to the patient can be determined for each drug, providing a clinician with an overview of how likely any particular drug is likely to cause the specific patient in consultation to experience side effects. This overall drug weighting score Dw can feed into the determination of whether a drug is suitable for a particular patient.

Although not preferred, it would be possible to use the overall drug weighting score Dw to determine whether to exclude particular drugs from being displayed in the list discussed above. For example, if Dw for a particular drug were to be larger than a predetermined threshold, that drug could be removed from the list. Instead, or as well, the overall drug weighting score Dw could be used to order the drugs in the list.

The 10 most prescribed drugs (for example for the hospital, clinic, region, healthcare provider, insurance provider, or country etc) for patients having the same diagnosis will be displayed in order of their respective overall drug weighting scores Dw. The overall drug weighting scores Dw may be compared to different thresholds to determine whether to display one or two exclamation marks next to them. Thus, the number of exclamation marks displayed next to each drug will vary between patients, although the drugs included in the list will not.

Only the most prescribed drugs are shown to avoid the clinician needing to look through a long list of drugs he is unlikely to prescribe. Naturally any number other than 10 may be shown. Moreover, the user may opt to view the full list of drugs, which will each be presented with no, one or two exclamation marks next to them in the same way.

In a preferred embodiment, in addition or instead, the results of each individual multiplication InWn are compared with one or more thresholds. If any one of them falls above a lower threshold, then the drug may be displayed with one exclamation mark and if any one of them falls above the upper threshold, the drug may be displayed with two exclamation marks.

Thus, as explained above, a single exclamation mark may be used to indicate that the drug should be used with some caution, as the patient's profile suggests that he may have an increased risk of one or more adverse side effects. Two exclamation marks may be used to indicate that the drug should be used with extreme caution, as the patient's profile suggests that he is likely to have an increased risk of one or more adverse side effects. Naturally, the number of thresholds and the number of exclamation marks (or other indicators) may vary.

Once the list of most prescribed drugs has been retrieved, it may be displayed in the order of drugs for which no value of InWn is above the lower threshold, in the sub-order of overall drug weighting score Dw; drugs for which one or more values of InWn is above the lower threshold, in the sub-order of the number of values InWn over the lower threshold and the sub-sub-order of overall drug weighting score Dw; and drugs for which one or more values of InWn is above the upper threshold, in the sub-order of the number of values InWn over the upper threshold, the sub-sub-order of the number of values InWn over the lower threshold and the sub-sub-sub-order of overall drug weighting score Dw. The user may also be able to command the user interface 280 to display all available drugs rather than just the top 10 or 5, and these may be displayed with exclamation marks in the same way. Optionally, the list of all the drugs may be re-ordered as discussed above.

As noted above, a clinician may select a side effect which the patient wishes to avoid. This may be fed back to the rules engine 270 to adjust the calculation described above. For example, the weightings W in the drug weightings table for the selected side effect and/or the patient's relative risk indicator may be multiplied by a predetermined coefficient to increase its significance in the value of InWn and Dw. Alternatively, the threshold(s) for the side effect may be lowered.

Accordingly, the drug table provides a wealth of information tailored to the specific patient to allow the clinician and the patient to discuss suitable AP drug treatment options and select an AP drug for treatment. In general existing practice the risk specific to a particular patient of experiencing side effects due to a drug has not previously been considered during patient consultations. By providing patient relative risk indicators and mapping them to drugs, the present invention allows clinicians to avoid a large amount of trial and error in finding the most suitable drugs to treat patients, thereby providing significantly improved outcomes in less time and saving healthcare providers significant sums of money.

The foregoing description has been given by way of example only and it will be appreciated by a person skilled in the art that modifications can be made without departing from the scope of the present invention.

For example, the foregoing description describes operation of the personalised medicine system 1, 100 by reference to application to the treatment of mental illness. However, the present invention is not limited to this and can be applied across the spectrum of medical complaints. Although applicable to general practice and any illness or condition, the present invention has particular benefit in the treatment of longer term illnesses and conditions, which require on-going treatment and many consultations.

Although the present invention has been described by reference to consultations and visits by the patient to the clinician, it should be understood that consultations need not be face to face (although this is preferred). Moreover, as discussed above, the personalised medicine system 1, 100 may make provision for "dummy" visits/consultations, for example involving contact of the patient with other medical professionals or for adding the results of lab tests and other investigations.

Where the words "step" and "stage" have been used in this specification, they should not be construed narrowly and, except where the context requires otherwise, they may be considered to be synonymous. Thus, the terms incorporate nodes and sub-nodes as described above. Thus, each page could be considered as being associated with a separate, individual stage or step in the workflow, with these stages or steps being grouped into nodes and higher level stages or steps.

## Claims

1. A personalised medicine system comprising:

   a processor;
   a memory;
   an input module; and
   an output module,
   said personalised medicine system using a first database of side effects associated with at least one predetermined condition and of moderators influencing a likelihood of experiencing said side effects, and being adapted:

      to receive an input of patient data of a patient; and
      based on the patient data and the first database, to determine for each side effect a first risk indicator of a risk that the patient will experience the side effect.

2. A personalised medicine system according to claim 1, wherein the database includes, for each of said moderators, at least one category associated with the moderator, and each side effect associated with each category.

3. A personalised medicine system according to claim 2, wherein the database further stores a strength value for each moderator and a category value for each category.

4. A personalised medicine system according to claim 3, wherein the determination of a said first risk indicator for a side effect comprises:

   establishing which categories influence a likelihood of experiencing the side effect;
for each established category, determining the categories in which the patient falls based on the patient data; and
combining the category value of each of the determined categories with the respective strength value of the corresponding moderator.

**5.** A personalised medicine system according to claim 3 or claim 4, wherein at least the category values for each moderator are normalised, whereby minimum and maximum allowed category values are the same for each moderator.

**6.** A personalised medicine system according to any one of the preceding claims, wherein the first risk indicator is an indication of a risk compared to a general population that the patient will experience the side effect.

**7.** A personalised medicine system according to any one of the preceding claims, further configured to display which moderators influence a likelihood of the patient experiencing a side effect.

**8.** A personalised medicine system according to any one of the preceding claims, being further configured to display the side effects in order of the values of the respective first risk indicators.

**9.** A personalised medicine system according to claim 8, further configured, on receipt of an input from a user, to store an indication that a patient wishes to avoid a displayed side effect.

**10.** A personalised medicine system according to any one of the preceding claims, further using a second database of side effects associated with each of a plurality of drugs and weightings influencing a likelihood of experiencing said effects for each drug, said system being adapted
based on the first risk indicators and the weightings, to determine for each drug a second risk indicator of a risk that the patient will experience side effects from taking the drug.

**11.** A personalised medicine system comprising:

a processor;
a memory;
an input module; and
an output module,
said personalised medicine system using:

for each of a plurality of side effects, a respective first indicator of a risk that a specific patient will experience the side effect; and
a table of side effects associated with each of a plurality of drugs and weightings influencing a likelihood of experiencing said effects for each drug,

and being adapted:

based on the first indicators and the weightings, to determine for each drug a second indicator of a risk that the patient will experience side effects from taking the drug.

**12.** A personalised medicine system according to claim 10 or claim 11, the second indicator for a drug being determined by combining the weighting for each respective side effect associated with the drug with first indicator for the corresponding side effect or corresponding to the highest combination of a weighting with a respective first risk indicator.

**13.** A personalised medicine system according to claim 12, the second risk indicator corresponding to a combination of respective combinations weightings with first risk indicators.

**14.** A personalised medicine system according to any one of claims 10 to 13, further configured to:

store an indication that the patient wishes to avoid a side effect; and
determine the second indicator based on the indication.

**15.** A personalised medicine system according to any one of claims 10 to 14, further configured to display a list of drugs for use by the patient, together with a representation of the likelihood a patient will suffer a side effect associated with the drug, wherein the representation is based on whether the second indicator for a drug is above a predetermined threshold.

# Fig. 1

1800

```
                PROCESSING                    MEMORY
                   UNIT                        1805
                   1806
```

BUS        1804

```
   GRAPHICS          NON-VOLATILE            I/O
  SUBSYSTEM            STORAGE           CONTROLLER
    1803                1808               1807
```

```
                                                          I/O
                                                        DEVICES
```

```
    DISPLAY
     1802
```

```
                                         DIGITAL IMAGE
                                         INPUT DEVICE
                                            1810
```

```
                MODEM OR
                NETWORK
                INTERFACE
                  1801
```

## Fig. 2

130

140

100

1, 110

120

# Fig. 3

| Workflow step | Workflow node | Page ID | Activity | Base |
|---|---|---|---|---|
| 1. Review Data | Electronic data exchange | P004 | Electronic data exchange | |
| | Review | P001 | Reviewing actions from last time | |
| | | P002 | View actions due this time | [KB01] |
| | | P003 | Reviewing any documentation from external sources | |
| 2. Assess Patient Status | Conduct clinical interview | P005 | Updating patient profile | [KB20] |
| | Review smartphone data | P007 | Reviewing smartphone data | |
| | Assess efficacy/examine mental state | P008 | Completing symptom rating scales | [KB03] |
| | | P009 | Recording symptom severity | |
| | Assess tolerability/physical state/side effects | P010 | Completing side effect rating scales | [KB05] |
| | | P011 | Doing a physical exam | |
| | | P012 | Recording side effect severity | |
| | Assess adherence | P013 | Reviewing Smartphone adherence data | |
| | | P013 | Evaluating and recording adherence | [KB06] |
| | Assess patient preferences | P014 | Understanding patient and carer options | [KB19] |
| 3. Formulate Management Plan | Review working diagnosis | P015 | Selecting a primary diagnosis and co-morbidities | [KB21] |
| | Determine pharmacological treatment | P016 | Deciding whether or not to change medication regimen and recording reasons for changes | [KB07] |
| | | P017 | Doctor determines how he wants to make changes to AP medication | |
| | | P018 | Using the treatment selection wizard to guide AP selection – view side effect relative risk | [KB08] |
| | | P019 | Viewing drug guidance matrix | [KB11] |
| | | P020 | Considering dug options | [KB12] |
| | | P028 | Selecting drugs | [KB22] |
| | | P021 | Confirming and prescribing drugs | |
| | Determine investigations | P022 | Determining investigations | [KB14] |
| | Order investigations | P022 | Ordering investigations | |
| | Determine non-pharmacological treatment | P023 | Selecting psychological interventions | [KB15] |
| | | P023 | Selecting social / lifestyle interventions | |
| | | P023 | Selecting medical interventions | |
| 4. Additional Patient Centred Interventions | Smartphone | P024 | Providing patient with smartphone app or updating it | |
| | Patient engagement and education | P025 | Providing patient with additional information / sources of support | [KB16] |
| | Homework | P025 | Providing patient with homework (3rd-party resources) | |
| 5. Wrap up | Next appointment | P026 | Set date of next appointment | [KB17] |
| | | P026 | Review reminders for the next appointment, add any others | |
| | Communication with healthcare professionals | P027 | Sharing information | [KB18] |
| | Data exchanges to EHR | P027 | Updating Electronic Health Record (if relevant) | |
| | Electronic data exchange | | Electronic data exchange | |

18

## Fig. 4

60

65

| John SMITH  ID#12345   DOB 10/15/1982 (30)   Male | Visit 08/20/2012 | | | | |
|---|---|

| | ⌄ Review Data | 〉 Assess Patient Status | 〉 Formulate Management Plan | 〉 Wrap-Up | Complete Visit |
|---|---|---|---|---|---|

### Review Data

| Electronic Data Exchange | Review | Interim Contacts |
|---|---|---|
| | Actions From Last Visit | External Documents |
| | Actions Due | Record Changes |
| | Open Monitoring Schedule | |

**Interim Contacts**

⌄  External Documents

Has anything changed since John Smith's last visit?
◉ unchanged     ○ changed

**Guided Recommendations**

⌄  Knowledge Base Recommendations

**NICE guidelines**

Labs                Lipid Profile (Last result on 06/05/2012)

P003

## Fig. 5

| ⌄   Actions From Last Visit | Date of Last Visit **07/20/2012** |
|---|---|
| **Antipyschotic Medication** | Risperidone 4mg od - Increased due to lack of efficacy |
| **Other Medication** | Fluoxetine 20mg od - commenced |
| **Labs** | Lipid<br>Glucose |
| **Physical** | BP **120/80**<br>Weight **57kg** |
| **Rating Scales** | PANSS<br>Total **79**<br>Positive symptoms **18**<br>Negative symptoms **13**<br>General Psychopathology **48** |
| **Remote Monitoring** | Adherence monitoring |
| **Non-pharmacological** | Not included this time as no activities last time |

P001

# Fig. 6

| | |
|---|---|
| **Actions Due** | Date Actions Due **08/20/2012** |
| **Labs** | Liver function test |
| **Physical** | Weight |
| **Rating Scales** | BPRS |
| **Non-pharmacological** | Set up art therapy class |

P002

# Fig. 7

| | |
|---|---|
| **Actions From Last Visit** | Date of Last Visit **07/20/2012** |
| **Antipyschotic Medication** | Risperidone 4mg od - Increased due to lack of efficacy |
| **Other Medication** | Fluoxetine 20mg od - commenced |
| **Labs** | Lipid<br>Glucose |
| **Physical** | BP 120/80<br>Weight 57kg |
| **Rating Scales** | PANSS<br>Total 79<br>Positive symptoms 18<br>Negative symptoms 13<br>General Psychopathology 48 |
| **Remote Monitoring** | Adherence monitoring |
| **Non-pharmacological** | Not included this time as no activities last time |

| | |
|---|---|
| **Actions Due** | Date Actions Due **08/20/2012** |
| **Labs** | Liver function test |
| **Physical** | Weight |
| **Rating Scales** | BPRS |
| **Non-pharmacological** | Set up art therapy class |

**Guided Recommendation**

| **Knowledge Base Recomm** | |
|---|---|
| **NICE guidelines** | |
| **Labs** | Lipid Profile (L |
| **Physical** | BP<br>Weight |
| **Rating Scales** | PANSS |

**mehealth™ Schizophreni**

**Labs** Glucose (Last result on 06/(

Consider repeating now on the SMITH's profile

**Family History** Diabetes
**Last Glucose** 105 mg/dl +

**Open Monitoring**

P001

P002

50

Conduct Clinical Interview

External Documents

In order for mehealth to take into account John Smith's personal profile and past treaments, please take time on first visit to work through the sections provided below and complete details of John Smith's profile.

Record Changes

| | | Document Quick Add |
|---|---|---|
| Diagnosis | Record Diagnosis | Quickly add any supporting documentation about John Smith, here. |
| Antipsychotic Treatments | Record Antipsychotic Medications | |
| Pyschotropic Treatments | Record Pyschotropic Medications | File name: |
| Contact | Record Contact(s) | |
| Rating Scales | Record Rating Scale(s) | |
| Physical Exams | Record Physical Exam(s) | |
| Lab Results | Record Lab Results | |
| Non-Pharmacological Teatments | Record Non-Pharmacological Treatments | |

Continue to John Smith's Profile >>

Guided Recommendations

Knowledge Base Recommendations

**NICE guidelines**

**Labs** — Lipid Profile (Last result on 06/05/2012)

**Physical** — BP
Weight

**Rating Scales** — PANSS

mehealth™ Schizophrenia Recommendations

**Labs** Glucose (Last result on 06/05/2012)

Consider repeating now on the basis of John SMITH's profile

**Family History** Diabetes
**Last Glucose** 105 mg/dl

**Open Monitoring Schedule**

50

Fig. 8

P003

# Fig. 9

**60** ) Review Data   ✓ Assess Patient Status   ) Formulate Management Plan   ) Wrap-Up   Complete Visit

**65**

Conduct Clinical Interview > **Patient Profile** > Examine Mental State > Assess Physical Status > Assess Adherence > Assess Patient Preferences

**50**

∨ Patient Profile

∨ Personal Details

| Ethnicity: | Asian: Chinese alone ⌄ |
| Alcohol: | No alcohol ⌄ |
| Smoking: | ○ Yes ⊙ No ○ Unknown |
| Illicit Drugs: | ○ Yes ⊙ No ○ Unknown |
| Height: | 67 |
| Weight: | 160 |

Edit

**Guided Recommendations**

∨ Knowledge Base Recommendations

∨ mehealth™ Schizophrenia Recommendations

∨ Psychiatric History

| Angina or myocardial infarction: | ⊙ Yes ○ No ○ Unknown |
| Bipolar disorder: | ⊙ Yes ○ No ○ Unknown |
| Depression: | ⊙ Yes ○ No ○ Unknown |
| Diabetes type 1: | ⊙ Yes ○ No ○ Unknown |
| Diabetes type 2: | ⊙ Yes ○ No ○ Unknown |
| Excess weight or obesity: | ⊙ Yes ○ No ○ Unknown |
| Hypertension: | ⊙ Yes ○ No ○ Unknown |
| Schizophrenia / Schizoaffective disorder: | ⊙ Yes ○ No ○ Unknown |
| Stroke or transient ischemic attacks: | ⊙ Yes ○ No ○ Unknown |

Edit

∨ Medical History

|  | Yes, Currently | Yes, Not currently | No | Unknown |
|---|---|---|---|---|
| Abnormal cardiac rhythm: | ⊙ Receiving treatment | ○ Receiving treatment | ○ No | ○ Unknown |
| Abnormal lipid profile: | ⊙ Receiving treatment | ○ Receiving treatment | ○ No | ○ Unknown |
| Agranulocytosis: | ⊙ Receiving treatment | ○ Receiving treatment | ○ No | ○ Unknown |
| Angina or myocardial infarction: | ⊙ Receiving treatment | ○ Receiving treatment | ○ No | ○ Unknown |
| Diabetes type 1: | ⊙ Receiving treatment | ○ Receiving treatment | ○ No | ○ Unknown |
| Diabetes type 2: | ⊙ Receiving treatment | ○ Receiving treatment | ○ No | ○ Unknown |
| Excess weight or obesity: | ⊙ Receiving treatment | ○ Receiving treatment | ○ No | ○ Unknown |
| Hypertension: | ⊙ Receiving treatment | ○ Receiving treatment | ○ No | ○ Unknown |
| Movement disorder: | ⊙ Receiving treatment | ○ Receiving treatment | ○ No | ○ Unknown |
| Seizures / Epilepsy: | ⊙ Receiving treatment | ○ Receiving treatment | ○ No | ○ Unknown |
| Stroke or transient ischemic attacks: | ⊙ Receiving treatment | ○ Receiving treatment | ○ No | ○ Unknown |

Edit

∨ Family History

|  | Yes, Currently | Yes, Not currently | No | Unknown |
|---|---|---|---|---|
| Anxiety: | ⊙ Receiving treatment | ○ Receiving treatment | ○ No | ○ Unknown |
| Depression: | ⊙ Receiving treatment | ○ Receiving treatment | ○ No | ○ Unknown |
| Manic episode: | ⊙ Receiving treatment | ○ Receiving treatment | ○ No | ○ Unknown |
| Sexual dysfunction: | ⊙ Receiving treatment | ○ Receiving treatment | ○ No | ○ Unknown |

Edit

P005

22

# Fig. 10

50

60

65

**Review Data** | ✓ Assess Patient Status | 〉 Formulate Management Plan | 〉 Wrap-

Conduct Clinical Interview > **Examine Mental State** > Assess Physical Status > Assess Adherence > Assess Patient Preferences

## Examine Mental State

∨ Symptom Rating Scales

To record symptoms you may use the rating scales below and/or select the Symptom Severity Mixer.

**∨ Complete Rating Scales**

Positive & Negative Symptoms

| PANSS | *Defer* | *View Past Results* |
| BPRS | *Defer* | *View Past Results* |

General Level of Functioning

| GAF | *Defer* | *View Past Results* |
| CGI | *Defer* | *View Past Results* |

Affective Symptoms

| CDSS | *Defer* | *View Past Results* |
| YMRS | *Defer* | *View Past Results* |

Side Effect Rating Scales >>

**∨ Past Rating Scale Results**

Guided Recor

∨ Knowledge

**NICE guideline**

Labs

**Physical**

**Rating Scales**

∨ mehealth'

**Labs** Glucose (L

Consider repeati
SMITH's profile

**Family History**
**Last Glucose**

Ope

⌃ Symptom Severity Mixer

<u>P008</u>

23

# Fig. 11

Positive & Negative Syndrome Scale
(PANSS)

Instructional text here .....

| Positive Scale (P) | Absent 0 | Minimal 1 | Mild 2 | Moderate Severe 3 | Moderate 4 | Severe 5 | Extreme 6 |
|---|---|---|---|---|---|---|---|
| **Delusions** Beliefs which are unfounded, unrealistic, and idiosyncratic. Basis for rating: Thought content expressed in the interview and its influence on social relations and behavior. | ● | ● | ● | ● | ● | ● | ● |
| **Conceptual disorganization** Disorganized process of thinking characterized by disruption of goal-directed sequencing, e.g., circumstantiality, tangentiality, loose associations, non sequiturs, gross illogicality, or though block. Basis for rating: Cognitive-verbal processes observed during the course of interview. | ● | ● | ● | ● | ● | ● | ● |
| **Excitement** Beliefs which are unfounded, unrealistic, and idiosyncratic. Basis for rating: Thought content expressed in the interview and its influence on social relations and behavior. | ● | ● | ● | ● | ● | ● | ● |

| Negative Scale (N) | Absent 0 | Minimal 1 | Mild 2 | Moderate Severe 3 | Moderate 4 | Severe 5 | Extreme 6 |
|---|---|---|---|---|---|---|---|
| **Blunted affect** Diminished emotional responsiveness as characterized by a reduction in facial expression, modulation of feelings, and communicative gestures. Basis for rating: observation of physical manifestations of affective tone and emotional responsiveness during the course of interview | ● | ● | ● | ● | ● | ● | ● |

# Fig. 12

75    72    74    70

Symptom Severity Mixer

**Positive Symptoms**
☐ adjust

**Negative Symptoms**
☐ adjust

**Cognition**
☐ adjust

**Suicidality**
☐ adjust

**Anxiety**
☑ adjust

**Depression**
☐ adjust

**Mania**
☐ adjust

76

P009

# Fig. 13

⌄ Side Effect Rating Scales

To record symptoms you may use the rating scales below and/or select the
Side Effect Severity Mixer.

⌄ Complete Rating Scales

AIMS    *Defer*

ACS    *Defer*

LUNSERS    *Defer*

Previous Results

P010

# Fig. 14

**65    60                                    50**

| John SMITH  ID#12345  DOB 10/15/1982 (30)  Male | Visit 08/20/2012 |

| ) Review Data | ~ Assess Patient Status | ) Formulate Management Plan | ) Wrap-Up | Complete Visit |

Conduct Clinical Interview > Patient Profile > Examine Mental State > **Assess Physical Status** > Assess Adherence > Assess Patient Preferences

## Assess Physical Status

∨ Physical Exam

Instructional text here ...

### Vital Signs

| | | | |
|---|---|---|---|
| Pulse | | bpm | Defer |
| Blood Pressure | | mm/Hg or cm/Hg | Defer |
| Temperature | | f or c | Defer |

### Body Weight & Height

| | | | |
|---|---|---|---|
| Body weight | | kg or lbs | Defer |
| Height | | feet/inches or m | Defer |
| BMI | | kg/m2 | Defer |

### Other Examinations

| | | |
|---|---|---|
| Eye examination | normal ∨ | Defer |
| Dental examinations | abnormal ∨ | Defer |

### Screenings

| | | |
|---|---|---|
| Symptoms of hyperprolactinemia | select ∨ | Defer |
| Abnormal involuntary movements | abnormal ∨ | Defer |
| Extrapyramidal side effects | normal ∨ | Defer |
| Extrapyramidal side effects, including akathisia | abnormal ∨ | Defer |

### Labs

| | | |
|---|---|---|
| ECG/EKG | select ∨ | Defer |
| EEG | abnormal ∨ | Defer |
| Imaging/EEG - CT | normal ∨ | Defer |
| Imaging/EEG - MRI | abnormal ∨ | Defer |

Save Physical Exam

∨ Physical Exam Results

### Guided Recommendations

∨ Knowledge Base Recommendations

∨ mehealth™ Schizophrenia Recommendations

P011

27

# Fig. 15

80

Side Effect Severity Sliders

**Weight Gain**

**Glucose Intolerance/Diabetes**

**Hyperlipidemia**

**Breast Enlargement/Galactorrhea**

**Amenorrhea/Dysmenorrhea**

**Other Sexual Side Effects**

**Seizures**

**Dystonia**

P012

## Fig. 16

60          65

John SMITH  ID#12345  DOB 10/15/1982 (30)  Male         Visit 08/20/2012

〉Review Data 〉Assess Patient Status ✓Formulate Management Plan 〉Wrap-Up  Complete Visit

**Working Diagnosis** > Pharmacological Treatment > Labs > Non-Pharmacological Treatment > Smartphone Data > Patient Engagement & Education

✓ Select Primary Diagnosis

Please select the current diagnosis by clicking the relevant button.

First episode psychosis   Schizophrenia

Affective psychosis   Non-affective psychosis

Manic   Mixed affective   Bipolar   Not bipolar

Save Primary Diagnosis 〉

✓ Select Co-morbidities

Please select any of the co-morbidities from the list below.

☐ Anxiety
☐ Sleep disturbance
☐ Substance abuse
☐ Alcohol abuse

Save Co-morbidities 〉

**Guided Recommendations**

✓ Knowledge Base Recommendations

✓ mehealth™ Schizophrenia Recommendations

P015          50

EP 2 782 064 A1

# Fig. 17

60    65

| John SMITH  ID#12345   DOB 10/15/1982 (30)   Male | | Visit 08/20/2012 | | | |

) Review Data  ›  ) Assess Patient Status  ›  ⌄ Formulate Management Plan  ›  ) Wrap-Up  ›  Complete Visit

**Working Diagnosis** > Pharmacological Treatment > Labs > Non Pharmacological Treatment > Smartphone Data > Patient Engagement & Education

⌄ Current Drug Treatment

Taking into account the efficacy, tolerability, adherence and patient preferences, please use the Treatment Configurator below to make any required changes or select "No Change" to continue with the John Smith's current treatment regimen.

Risperidone, 4mg od
Chlorpromazine, 200mg od
Antidepressant, 50mg od

No Change

⌄ Change Drug Treatment Selection

Please add, remove, review and complete drug treatment selection below.

**Selected Antipsychotics:**

*i* Risperidone   4 mg ▼   od ▼   oral ▼   discontinue
+ Add Antipsychotic

**Selected Psychotropic Drugs:**

*i* Chlorpromazine   200 mg ▼   od ▼   oral ▼   discontinue
+ Add Psychotropic

**Selected Other Drugs:**

*i* Antidepressant   50 mg ▼   od ▼   oral ▼   discontinue
+ Add Other

Save Treatment Selection

⌃ Treatment Selection Wizard

**Guided Recommendations**

⌄ Knowledge Base Recommendations

⌄ mehealth™ Schizophrenia Recommendations

68    P016    50

30

## Fig. 18

**^ SIDE EFFECT RELATIVE RISK**

Saeed Choudry's side effect risk profile has been analyzed based on the information you provided.
- Hover or click on each side effect for details.
- Risk factors have been identified from an expert panel review of the evidence base. Click the 🛈 to explore the relevant evidence base.
- Side effects are ranked according to this patient's risk relative to other patients.
- Characteristics which increase this patient's risk are detailed.

🛈 **Dystonia**

🛈 **Agranulocytosis**

🛈 **Hyperlipidemia**

🛈 **Weight Gain**

🛈 Parkinsonism

🛈 Sedation

🛈 Akathisia

🛈 Glucose Intolerance/Diabetes ⊗

Please select the side effects you would like to avoid.
For example, akathisia may be particularly undesirable in a patient who is agitated.

P018

31

EP 2 782 064 A1

# Fig. 19

∨ Drug Selection Wizard

Taking into account **John Smith's** side effect risk and symptoms the drug selection wizard has matched these 10 most commonly prescribed oral and injectable antipsychotics.

**Discuss Selected Drugs** >

## ∨ Top 10 Antipsychotics

Customized Oral List

| Drug Name | NICE | United Healthcare | Maudsley | Mass Health | Drug Info | Cost |
|---|---|---|---|---|---|---|
| ☐ quetiapine | ● | ● | ● | | ⓘ | $$ |
| ■ aripiprazole | ● | ● | ● | | ⓘ | $$ |
| ☐ risperidone | ● | ● | ● | ● | ⓘ | $$ |
| ■ olanzapine | ● | ● | ● | | ⓘ | $ |
| ☐ clozapine | | | | | ⓘ | $$ |
| ■ asenapine | ● | ● | ● | | ⓘ | $ |
| ☐ ziprasidone | ● | ● | ● | | ⓘ | $ |
| ■ fluphenazine | ● | | | | ⓘ | $$ |
| ☐ lurasidone | ● | ● | ● | | ⓘ | $$ |
| ■ perphenazine | ● | | | | ⓘ | $$$ |

Customized Injectable List

| Drug Name | NICE | United Healthcare | Maudsley | Mass Health | Drug Info | Cost |
|---|---|---|---|---|---|---|
| ☐ haloperidol | | | | | ⓘ | $$ |
| ■ flupentixol | | | | | ⓘ | $$ |
| ☐ risperidone | | | | | ⓘ | $$ |
| ■ zuclopenthixol | | | | | ⓘ | $$ |

P019

32

EP 2 782 064 A1

## Fig. 20A

**410**    **432**

| Visit | Anitpsychotic Medication | Other Medications | Physical | Labs | Rating Scales | Mobile Applications | Non Pharmacological Interventions |
|---|---|---|---|---|---|---|---|
| **Start Visit** ⇥ | ⇥ | ⇥ | ⇥ | ⇥ | ⇥ | ⇥ | ⇥ |
| 10-Jul-12 | Risperdone 4 mg od | Fluxetine 4 mg od | ▼ BP / ▲ Weight | ► Lipids / ▼ Glucose | | Adherence Monitoring | Art Therapy |
| 29-Jun-12 | | | ► Extrapyrmidal Side Effects | EKG | ▲ BPRS / ▼ GAF | | |
| 18-May-12 | Olanzapine 10 mg od | | | ► Glucose | ► PANSS | | |
| 30-Dec-11 | Seroquel 4 mg od | | | ► Glucose | ► CDSS | | |

**420**    **425**    **400**    **430**    **445**

33

# Fig. 20B

Select Visit Type

Select Visit Date

2/26/2013

Start >

# Fig. 20C

| Visit | Anitpsychotic Medication | Other Medications | Physical | Labs | Rating Scales | Mobil Applicati |
|---|---|---|---|---|---|---|
| Start | →I | →I | →I | →I | →I | |
| 10-Jul-12 | Risperdone 4 mg od | Fluxetine 4 mg od | ▼ BP / ▲ Weight | ► Lipids / ▼ Glucose | | Adhere Monitor |
| 29-Jun-12 | | | ► Screening: Extrapyrmidal Side Effects | ▲ EKG | ▲ BPRS / ▼ GAF | |
| 18-May-12 | Olanzapine 10 mg od | | | ► EKG | ► PANSS | |
| 30-Dec-11 | Seroquel 4 mg od | | | ► EKG | ► CDSS | |

470

400

# Fig. 21

Alan BROWN ID#00014 DOB 02/08/1993 (19) Male

∨ Review Data 〉 Patient Status 〉 Formulate Management Plan 〉 Wrap-Up 〉 Complete Visit

Data Exchange > Review

∨ Actions from Last Visit Date of Last Visit

∧ Actions Due

∧ Documentation

Guided Recommendations

〉 Expand

| Visit | Anitpsychotic Medication | Other Medications | Physical | Labs | Rating Scales | Mobile Applications |
|---|---|---|---|---|---|---|
| 28-Aug-12 | Drugname 4 md od | Drugname dose | Pulse / BMI | | PANSS | |

447 445

450

# Fig. 22

## Fig. 23

| Moderators of outcome/Risk factors | Variable categories | WG | AGRAN | AKATH | DIAB | TD | DYSLIPID |
|---|---|---|---|---|---|---|---|
| Age | <19 | y | | | | | y |
| | 20 to 30 | y | | | | | |
| | 31 to 45 | y | | | | | |
| | 45 to 64 | y | | | | | |
| | <21 | | y | | | | |
| | <40 | | | | | y | |
| | >40 | | y | | | y | |
| | >50 | | y | y | | | |
| | >60 | | y | | | y | |
| | <50 | | | y | | | |
| | <45 | | | | y | y | y |
| | > or = 45 | | | | y | | y |
| | ≥55 | | | | | | Y |
| | <55 | | | | | | Y |
| | 25 to 60 | | | | | y | |
| Gender | Male | y | y | y | y | y | y |
| | Female | y | y | y | y | y | y |
| Race | White alone | y | y | | y | y | |
| | non white/ non Caucasian | y | | | y | y | |
| | african – American | y | | | | | |
| | Hispanic | y | | | | | |
| | Indian | y | | | | | |
| | Pakistani | y | | | | | |
| | Chinese | y | | | | | |
| | Japanese | y | | | | | |
| | Other | y | | | | | |
| | Asian | | y | | | | |
| | African- Caribbean | | y | | | | |
| Acculturation (adoption of another culture) | Acculturation | | | y | | y | |
| | Maintenance of own culture | | | y | | y | |
| Marital status | Married | | | | | y | |
| | Single | | | | | y | |
| Employment status | Employed | | | | | y | |
| | not employed | | | | | y | |
| Smoking | Smoker | y | | | y | y | y |
| | Non-smoker | y | | | y | y | y |
| Alcohol | No alcohol | y | | | y | | |
| | Occasional | y | | | | | |
| | alcohol abuse | y | | | | y | |
| | Diagnosed alcohol dependence | y | | | | | |
| Alcohol intake | Heavy consumption or binge drinking | | | | y | | |
| | Light/moderate consumption (<30units a week) | | | | y | | |
| Acute alcohol intake | Acute intake >8 units | | | y | | | |
| | Lower acute intake | | | y | | | |
| Alcohol consumption | excessive (> 21 male/14 female units) | | | | | | Y |
| | units within recommended range | | | | | | Y |
| Psychoactive substances | Use of cocaine | | | y | | | |
| | Other | | | y | | | |
| Cannabis usage | Yes | y | | | | | |
| | No | y | | | | | |
| Drug usage | Yes | | | | | y | |
| | No | | | | | y | |

# Fig. 24

# WEIGHT GAIN INFLUENCES

| Moderator of outcome | Moderator Strength (Importance of moderator in overall prediction) | Category | Category Strength (Specific influence of variables within each moderator) | References |
|---|---|---|---|---|
| Age | 3 | <19 | 3 | 2 |
| | | 20 to 30 | 2 | 3 |
| | | 31 to 45 | 1 | 6 |
| | | 46 to 64 | 1 | 7 |
| | | >65 | 1 | 12 |
| | | | | 16 |
| Gender | 2 | male | 2 | 4 |
| | | female | 1 | 3 |
| | | | | 17 |
| | | | | 21 |
| Race | 3 | white alone | 2 | 3 |
| | | non white; | 3 | 22 |
| | | african - american | 2 | 26 |
| | | hispanic | 1 | 30 |
| | | indian | 1 | 43 |
| | | pakistani | 1 | 5 |
| | | chinese | no data | 44 |
| | | japanese | no data | |
| | | pima indians living in Arizona | 1 | 19 |
| | | pima indians living in Mexico | | |
| | | other | | |
| Smoking | 1 | Smoker | 2 | 14 |
| | | Non-smoker | 1 | |
| | | | | 19 |
| Alcohol | 1 | No alcohol | 1 | 26 |
| | | Occasional | 1 | |
| | | Alcohol abuse | 2 | |
| | | Diagnosed alcohol dependence | no data | |
| Cannabis usage | 2 | Yes | 2 | 22 |
| | | No | 1 | |
| INCREASED appetite? * This moderator occurs after Tx with AP * | 2 | Increased appetite | 3 | 3 |
| | | No increaased appetite | 1 | 11 |
| | | | | 19 |
| | | | | 24 |
| poor diet | 1 | Poor diet | 2 | 6 |
| | | Good diet | 1 | 16 |
| | | | | 22 |
| lack of exercise | 2 | Lack of exercise | 2 | 6 |
| | | Regular exercise | 1 | 16 |
| | | | | 22 |
| | | | | 31 |
| low levels of self control | 1 | Low self control | 2 | 11 |
| | | Higher self control | 1 | 22 |

# Fig. 25

## DRUG SIDE EFFECTS WEIGHTING TABLE

| Side Effect | Olanzapine | Risperidone | Amisulpride | Quetiapine | Aripiprazole | Clozapine |
|---|---|---|---|---|---|---|
| Hyperlipidemia | 2 | 3 | 1 | 5 | 4 | 2 |
| Breast Enlargement/ Galactorrhoea | 3 | 2 | 2 | 2 | 2 | 3 |
| Weight Gain | 5 | 3 | 1 | 3 | 2 | 5 |
| Glucose Intolerance/Diabetes | 5 | 3 | 2 | 3 | 1 | 5 |
| Amenorrhoea/ Dysmenorrhoea | 5 | 3 | 2 | 4 | 5 | 1 |
| Other Sexual Side Effects | 2 | 3 | 5 | 2 | 3 | 2 |
| Sedation | 3 | 1 | 4 | 1 | 3 | 3 |
| Hypotension | 1 | 5 | 3 | 5 | 1 | 1 |
| Seizures | 3 | 3 | 1 | 4 | 4 | 3 |
| Dystonia | 2 | 4 | 2 | 3 | 3 | 5 |
| Anticholingeric Side Effects | 5 | 4 | 3 | 2 | 1 | 4 |

## EUROPEAN SEARCH REPORT

Application Number

EP 13 15 9809

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| L | EPO: "Mitteilung des Europäischen Patentamts vom 1. Oktober 2007 über Geschäftsmethoden = Notice from the European Patent Office dated 1 October 2007 concerning business methods = Communiqué de l'Office européen des brevets,en date du 1er octobre 2007, concernant les méthodes dans le domaine des activités", JOURNAL OFFICIEL DE L'OFFICE EUROPEEN DES BREVETS.OFFICIAL JOURNAL OF THE EUROPEAN PATENT OFFICE.AMTSBLATTT DES EUROPAEISCHEN PATENTAMTS, OEB, MUNCHEN, DE, vol. 30, no. 11, 1 November 2007 (2007-11-01), pages 592-593, XP007905525, ISSN: 0170-9291 * Statement in accordance with the Notice from the European Patent Office dated 1 October 2007 concerning business methods (OJ 11/2007; p592f) The claimed subject matter, with due regard to the description and drawings, relates to processes comprised in the list of subject matter and activities excluded from patentability under Art.52(2) and (3) EPC. The applicant is advised that in accordance with the established practice of the EPO, no search need be performed in respect of those aspects of the claimed invention. The only identifiable technical aspects of the claimed invention relate to the use of conventional, general-purpose data processing technology for processing data of an inherently non-technical nature. The information technology employed is considered to have been generally known as it was widely available to everyone at the date of filing/priority of the present | 1-15 | INV. G06Q50/24 |
| | | | TECHNICAL FIELDS SEARCHED (IPC) |
| | | | G06Q |

The present search report has been drawn up for all claims

| Place of search | Date of completion of the search | Examiner |
|---|---|---|
| The Hague | 25 July 2013 | Tiago Pinheiro |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

# EUROPEAN SEARCH REPORT

**Application Number**

EP 13 15 9809

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | application. The notoriety of such prior art cannot reasonably be contested. No documentary evidence was therefore considered required. *<br><br>----- | | |
| | | | **TECHNICAL FIELDS SEARCHED (IPC)** |

The present search report has been drawn up for all claims

| Place of search | Date of completion of the search | Examiner |
|---|---|---|
| The Hague | 25 July 2013 | Tiago Pinheiro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................................

& : member of the same patent family, corresponding document

EPO FORM 1503 03.82 (P04C01)